# EUROPEAN PATENT SPECIFICATION

(11) **EP 4 511 077 B1**
(45) Date of publication and mention of the grant of the patent: **18.11.2026**
(21) Application number: 23722778.0
(22) Date of filing: 18.04.2023
(51) Int. Cl.: A61L 27/18

(54) **BONE REPAIR COMPOSITIONS COMPRISING POLYTHIOURETHANE AND USES THEREOF**
POLYTHIOURETHAN ENTHALTENDE KNOCHENREPARATURZUSAMMENSETZUNGEN UND IHRE VERWENDUNG
COMPOSITIONS DE RÉPARATION OSSEUSE COMPRENANT DU POLYTHIOURÉTHANE ET LEURS UTILISATIONS

(30) Priority: 20.04.2022 US 202263332845 P; 12.09.2022 US 202263375281 P
(43) Date of publication of application: 26.02.2025
(73) Proprietor: Abyrx, Inc., Stamford, CT 06902 (US)
(72) Inventor: JOVANOVIC, Aleksa, MEMPHIS, Tennessee 38116 (US); BEARDEN, Robert, MEMPHIS, Tennessee 38116 (US); ROSE, John, MEMPHIS, Tennessee 38116 (US); MILLER, Kirsten, Fort Worth, Texas 76109 (US)
(74) Representative: Haseltine Lake Kempner LLP
(86) International application number: PCT/US2023/018936
(87) International publication number: WO 2023/205141

(56) References cited:
- CN-A- 114 058 314
- US-A1- 2002 125 410
- US-A1- 2017 119 924
- BONZANI ET AL: "Synthesis of two-component injectable polyurethanes for bone tissue engineering", BIOMATERIALS, ELSEVIER, AMSTERDAM, NL, vol. 28, no. 3, 1 January 2007 (2007-01-01), pages 423 - 433, XP005717761, ISSN: 0142-9612, DOI: 10.1016/J.BIOMATERIALS.2006.08.026

## Description

### CROSS REFERENCE TO RELATED APPLICATIONS

This application claims priority to and the benefit of priority of U.S. Provisional Application No. 63/332,845, filed April 20, 2022, and U.S. Provisional Application No. 63/375,281, filed September 12, 2022.

### FIELD OF THE INVENTION

The present disclosure generally relates to compositions and methods in the field of bone void fillers, bone graft substitutes, and/or bone cements/adhesives for use in the repair and reconstruction of bone and bone defects.

### BACKGROUND

Bone repair compositions currently available in the medical field, such as those described in U.S. Pat. App. Pub. Nos. 2018/0344898 and 2022/0241457, generally utilize isocyanate reaction chemistry with polyols to produce polyurethane compositions that cure to a hard solid form. However, the catalysts that are used in polyurethane chemistry are generally sensitive to moisture, oxygen, temperature, and/or pH, which can affect the shelf-life stability of these products. Isocyanate reaction chemistry with thiols produce polythiourethane compositions that also cure to a hard solid form. However, these polythiourethane compositions are generally not used in the medical field because of their quick curing times. These quick curing times are substantially instantaneous reactions that do not provide ample time for a user to administer the polythiourethane to a subject before it cures to a hard solid. Additionally, despite attempts to standardize mixing during reaction of isocyanate with thiol, variation in users' mixing techniques, for example, can complicate the provision of instructions for mixing. Assessment of the degree to which the isocyanate has mixed with the thiol has also been challenging. US 2017/119924 A1 describes a biodegradable scaffold, a low-molecular weight thioketal, and a method of forming a biodegradable scaffold.

### SUMMARY

The scope of the invention is defined by the claims.

The present disclosure provides a solution to at least one or more of the aforementioned limitations and deficiencies in the art generally relating to bone repair compositions. In one aspect, a solution is premised on the use of compositions comprising two separate reactive components which can be mixed together to form a polythiourethane, the first component comprising isocyanates and the second component comprising thiols. The first component and/or the second component can further comprise a colorant to provide a visual indicator that the first component and the second component have been sufficiently mixed (e.g., mixed to homogeneity such as, for example, the composition has a generally consistent color). The compositions disclosed herein surprisingly had curing times that would provide sufficient time for a user to administer the polythiourethane to a subject before it cured to a hard solid form. An advantage of the compositions and processes of the present invention is the shelf-life stability of the products (e.g., reliance on environmentally sensitive catalysts can be reduced or avoided all together). Another advantage is the polythiourethane-based compositions provide a sufficient amount of time for administration to the subject while also providing a relatively quick curing time when compared with isocyanate/polyol-based polyurethane compositions. That is, there is a sufficient amount of time to apply the compositions of the present disclosure and a relatively quick waiting time to ensure that the compositions are set in place/cured.

In one aspect of the disclosure, disclosed is a composition comprising a bone repair composition comprising two separate reactive components that can be mixed together to form a polythiourethane: (a) the first component comprising: (i) one or more isocyanates having at least two functional isocyanate groups, and/or (ii) one or more isocyanate-terminated prepolymers comprising one or more isocyanates having at least two functional isocyanate groups; and (b) the second component comprising: (i) one or more thiols having at least two functional thiol groups, and/or (ii) one or more thiol-based prepolymers comprising one or more thiols having at least two functional thiol groups, wherein upon mixing (a) and (b), the functional thiol groups of the second component react with the functional isocyanate groups of the first component, thereby forming the polythiourethane, wherein the resulting polythiourethane cures into a solid form after the reaction is complete, and wherein the resulting polythiourethane is biocompatible and/or bioresorbable in bone. In some aspects, the polythiourethane is suitable for use as a bone cement, bone adhesive, bone void filler, bone graft substitute, and/or bone hemostatic agent.

In some aspects, the one or more isocyanates comprise methyl-2,6-diisocyanato hexanoate, ethyl-2,6-diisocyanato hexanoate, isophorone diisocyanate, toluene diisocyanate, 1,4-butane diisocyanate, 4,4'-methylene diphenyl diisocyanate, hexamethylene diisocyanate, 2,2,4-trimethyl hexamethylene diisocyanate, cyclohexyl methylene diisocyanate, methylene-bis(4-cyclohexylisocyanate), 1,4-cyclohexane diisocyanate, tetraethyl methylene diphenyl diisocyanate, dianisidine diisocyanate, m-tetramethyl xylene diisocyanate, p-tetramethyl xylene diisocyanate, 1,8-diisocyanatooctane, 1,10-diisocyanatodecane, 1,12-diisocyanatododecane, lysine diisocyanate, lysine triisocyanate, 4,4'-methylenebis(cyclohexyl isocyanate), poly(hexamethylene diisocyanate), poly(propylene glycol) tolylene 2,4 diisocyanate, or combinations thereof. In some aspects, the one or more thiols comprise methanethiol, ethanethiol, 1-propanethiol, 2-propanethiol, 1,3-propanedithiol, 2-propenethiol, butanethiol, tert-butyl mercaptan, pentanethiol, thiophenol, 1-hexadecanethiol, pentachlorobenzenethiol, pentaerythritol tetrakis(3-mercaptoproprionate), trimethylolpropane tris(3-mercaptopropionate), HS-PEG1500-SH, 4arm-PEG10K-SH, 4arm-PEG15K-SH, 4arm-PEG20K-SH, or combinations thereof. In some aspects, the one or more thiols comprise 1,3-propanedithiol, trimethylolpropane tris(3-mercaptopropionate), pentaerythritol tetrakis(3-mercaptoproprionate), or combinations thereof. In some aspects, the one or more isocyanates comprise 4,4'-methylenebis(cyclohexyl isocyanate), poly(hexamethylene diisocyanate), poly(propylene glycol) tolylene 2,4 diisocyanate, hexamethylene diisocyanate, isophorone diisocyanate, or combinations thereof.

In some aspects, the first component comprises one or more isocyanate-terminated prepolymers. In some aspects, the isocyanate-terminated prepolymers are the reaction product of: (a) an excess of the one or more isocyanates; and (b) (i) one or more polyols having at least two hydroxyl groups, and/or (ii) one or more thiols having at least two thiol groups. In some aspects, the isocyanate-terminated prepolymers do not have functional hydroxyl groups. In some aspects, the one or more isocyanates comprise hexamethylene diisocyanate or isophorone diisocyanate, or combinations thereof. In some aspects, the polyol is pentaerythritol. In some aspects, the one or more thiols comprise methanethiol, ethanethiol, 1-propanethiol, 2-propanethiol, 1,3-propanedithiol, 2-propenethiol, butanethiol, tert-butyl mercaptan, pentanethiol, thiophenol, 1-hexadecanethiol, pentachlorobenzenethiol, pentaerythritol tetrakis(3-mercaptoproprionate), trimethylolpropane tris(3-mercaptopropionate), HS-PEG1500-SH, 4arm-PEG10K-SH, 4arm-PEG15K-SH, 4arm-PEG20K-SH, or combinations thereof.

In some aspects, the second component comprises one or more thiol-based prepolymers. In some aspects, the thiol-based prepolymers are the reaction product of: (a) an excess of the one or more thiols; and (b) one or more isocyanates having at least two functional isocyanate groups. In some aspects, the one of more thiols comprise methanethiol, ethanethiol, 1-propanethiol, 2-propanethiol, 1,3-propanedithiol, 2-propenethiol, butanethiol, tert-butyl mercaptan, pentanethiol, thiophenol, 1-hexadecanethiol, pentachlorobenzenethiol, pentaerythritol tetrakis(3-mercaptoproprionate), trimethylolpropane tris(3-mercaptopropionate), HS-PEG1500-SH, 4arm-PEG10K-SH, 4arm-PEG15K-SH, 4arm-PEG20K-SH, or combinations thereof. In some aspects, the one or more isocyanates comprise methyl-2,6-diisocyanato hexanoate, ethyl-2,6-diisocyanato hexanoate, toluene diisocyanate, 1,4-butane diisocyanate, 4,4'-methylene diphenyl diisocyanate, 2,2,4-trimethyl hexamethylene diisocyanate, cyclohexyl methylene diisocyanate, methylene-bis(4-cyclohexylisocyanate), 1,4-cyclohexane diisocyanate, tetraethyl methylene diphenyl diisocyanate, dianisidine diisocyanate, m-tetramethyl xylene diisocyanate, p-tetramethyl xylene diisocyanate, 1,8-diisocyanatooctane, 1,10-diisocyanatodecane, 1,12-diisocyanatododecane, lysine diisocyanate, lysine triisocyanate, 4,4'-methylenebis(cyclohexyl isocyanate), poly(hexamethylene diisocyanate), poly(propylene glycol) tolylene 2,4 diisocyanate, hexamethylene diisocyanate, isophorone diisocyanate, or combinations thereof. In some aspects, the one or more thiols comprise 1,3-propanedithiol, trimethylolpropane tris(3-mercaptopropionate), pentaerythritol tetrakis(3-mercaptoproprionate), or combinations thereof. In some aspects, the one or more isocyanates comprise 4,4'-methylenebis(cyclohexyl isocyanate), poly(hexamethylene diisocyanate), poly(propylene glycol) tolylene 2,4 diisocyanate, hexamethylene diisocyanate, isophorone diisocyanate, or combinations thereof.

In some aspects, the compositions disclosed herein are not derived from or made from reacting an isocyanate with a polyol. In some aspects, the first component and/or the second component does not include a polyol, polyamine, and/or acrylate.

In some aspects, the first component and/or the second component are moldable putties. In some aspects, upon mixing of (a) and (b), the resulting polythiourethane is a moldable putty prior to curing. In some aspects, the compositions disclosed herein anhydrous. In some aspects, the compositions disclosed herein sterile.

In some aspects, the first component and/or second component further comprises one or more catalysts. In some aspects, the one or more catalysts comprise triethylamine, tributylamine, 1,8-diazabicyclo[5.4.0]undec-7-ene, dibutyltin dilaurate, stannous octoate, 1,4-diazabicyclo[2.2.2] octane, tetra n-butyl titanate, titanium acetylacetonate, triethanolamine titanate, titanium ethylaceto-acetate, tetraethyl titanate, tetraisopropyl titanate, titanium lactic acid chelate, triethylene diamine, bis(dimethylaminoethyl)ether, dimethylethanolamine, or combinations thereof. In certain aspects, the one or more catalysts comprise triethylamine. In certain aspects, the one or more catalysts comprise tributylamine.

In some aspects, the first component and/or the second component further comprises one or more particulate inorganic mineral compounds. In some aspects, the one or more particulate inorganic mineral compounds comprise aragonite, dahlite, calcite, calcium carbonate, amorphous calcium carbonate, vaterite, weddellite, whewellite, struvite, urate, ferrihydrite, francolite, monohydrocalcite, magnetite, goethite, dentin, calcium carbonate, calcium sulfate, calcium phosphosilicate, sodium phosphate, calcium aluminate, calcium phosphate, hydroxyapatite, α-tricalcium phosphate, β-tricalcium phosphate, dicalcium phosphate, 3-tricalcium phosphate, tetracalcium phosphate, amorphous calcium phosphate, octacalcium phosphate, calcium phosphate silica glass, fluoroapatite, chloroapatite, magnesium-substituted tricalcium phosphate, magnesium oxide, magnesium potassium phosphate, silica, silicon dioxide, soluble glass, sodium silicate, carbonate hydroxyapatite, or combinations thereof. In certain aspects, the one or more particulate inorganic mineral compounds comprise β-tricalcium phosphate. In certain aspects, the one or more particulate inorganic mineral compounds comprise hydroxyapatite. In certain aspects, the one or more particulate inorganic mineral compounds comprise silicon dioxide. In certain aspects, the one or more particulate inorganic mineral compounds comprise β-tricalcium phosphate and hydroxyapatite. In certain aspects, the one or more particulate inorganic mineral compounds comprise β-tricalcium phosphate and silicon dioxide. In some aspects, the one or more particulate inorganic mineral compounds (e.g., β-tricalcium phosphate, hydroxyapatite, and/or silicon dioxide) has a particle size of from about 5 microns to about 2500 microns.

In some aspects, the first component and/or the second component further comprises one or more plasticizers. In some aspects, the one or more plasticizers comprise polyethylene glycol diesters of stearic acid, petrolatum, white petrolatum, lanolin, PEG-6000 distearate, PEG-150 distearate, triacetin, poly(trimethylenecarbonate) (PTMC), polymethylmetacrylate (PMMA), polyvinylpyrrolidone (PVP), succinic anhydride, maleic anhydride, phthalic anhydride, or combinations thereof. In certain aspects, the one or more plasticizers comprise PEG-6000 distearate and/or triacetin. In certain aspects, the one or more plasticizers comprise PVP.

In some aspects, the first component and/or the second component further comprises one or more hydrocarbons. In some aspects, the one or more hydrocarbons comprise petrolatum, white petrolatum, or a combination thereof. In certain aspects, the one or more hydrocarbons comprise white petrolatum.

In some aspects, the first component and/or the second component further comprises one or more carbonates. In some aspects, the one or more carbonates comprise poly(trimethylene carbonate), calcium carbonate, magnesium carbonate, aluminum carbonate, iron carbonate, zinc carbonate, calcium bicarbonate, sodium bicarbonate, magnesium bicarbonate, aluminum bicarbonate, iron bicarbonate, zinc bicarbonate, or combinations thereof.

In some aspects, the first component and/or the second component further comprises one or more porogens and/or one or more chain extenders. In some aspects, the first component and/or the second component further comprises one or more antibiotics and/or one or more growth factors.

In some aspects, the first component and/or the second component further comprises a colorant. In some aspects, the first component comprises a colorant. In some aspects, the second component comprises a colorant. In some aspects, the first component and the second component comprise a colorant. In some aspects, the first component comprises a first colorant, and wherein the second component does not comprise a colorant or comprises a second colorant that is different than the first colorant. In some aspects, the second component comprises a first colorant, and wherein the first component does not comprise a colorant or comprises a second colorant that is different than the first colorant. In some aspects, the polythiourethane resulting from mixture of the first component and the second component comprises a color that is different from a color of the first component and/or a color of the second component. In some aspects, the colorant comprises D&C Violet No. 2, D&C Blue No. 6, and/or D&C Green No. 6. In certain aspects, the colorant comprises D&C Green No. 6. , the colorant comprises D&C Violet No. 2, D&C Blue No. 6, and/or D&C Green No. 6. In some aspects, the colorant comprises about 0.0001% to about 0.01% w/w (e.g., at least, at most, exactly, or between (inclusive or exclusive) of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, or 0.01% w/w) of the total amount of the first component, the total amount of the second component, the total amount of the first component and the total amount of the second component, or the total amount of the combination of the first component and the second component.

In certain aspects,: the first component comprises: an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst; pentaerythritol tetrakis(3-mercaptopropionate); tributylamine; white petrolatum; and β-tricalcium phosphate; and the second component comprises: isophorone diisocyanate; pentaerythritol tetrakis(3-mercaptopropionate); tributylamine; white petrolatum; and β-tricalcium phosphate. In certain aspects, the first component comprises: 35 to 45% (e.g., at least, at most, exactly, or between (inclusive or exclusive) of 5%, 36%, 37%, 38%, 39%, 40%, 41%, 42%, 43%, 44%, or 45%) by weight of an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst; 1 to 10% (e.g., at least, at most, exactly, or between (inclusive or exclusive) of 1%, 2%, 3%, 4%, 5%, 6%, 7%, 8%, 9%, or 10%) by weight of pentaerythritol tetrakis(3-mercaptopropionate); 0.00001 to 0.01% (e.g., at least, at most, exactly, or between (inclusive or exclusive) of 0.00001%, 0.00002%, 0.00003%, 0.00004%, 0.00005%, 0.00006%, 0.00007%, 0.00008%, 0.00009%, 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, or 0.01%) by weight of tributylamine; 0.1 to 5% (e.g., at least, at most, exactly, or between (inclusive or exclusive) of 0.1%, 0.2%, 0.3%, 0.4%, 0.5%, 0.6%, 0.7%, 0.8%, 0.9%, 1%, 2%, 3%, 4%, or 5%) by weight of white petrolatum; and 50 to 60% (e.g., at least, at most, exactly, or between (inclusive or exclusive) of 50%, 51%, 52%, 53%, 54%, 55%, 56%, 57%, 58%, 59%, or 60%) by weight of β-tricalcium phosphate; and the second component comprises: 1 to 10% (e.g., at least, at most, exactly, or between (inclusive or exclusive) of 1%, 2%, 3%, 4%, 5%, 6%, 7%, 8%, 9%, or 10%) by weight of isophorone diisocyanate; 30 to 40% (e.g., at least, at most, exactly, or between (inclusive or exclusive) of 30%, 31%, 32%, 33%, 34%, 35%, 36%, 37%, 38%, 39%, or 40%) by weight of pentaerythritol tetrakis(3-mercaptopropionate); 0.00001 to 0.01% (e.g., at least, at most, exactly, or between (inclusive or exclusive) of 0.00001%, 0.00002%, 0.00003%, 0.00004%, 0.00005%, 0.00006%, 0.00007%, 0.00008%, 0.00009%, 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, or 0.01%) by weight of tributylamine; 1 to 10% (e.g., at least, at most, exactly, or between (inclusive or exclusive) of 1%, 2%, 3%, 4%, 5%, 6%, 7%, 8%, 9%, or 10%) by weight of white petrolatum; and 50 to 60% by weight of β-tricalcium phosphate.

In certain aspects, the first component comprises: an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst; pentaerythritol tetrakis(3-mercaptopropionate); tributylamine; white petrolatum; and β-tricalcium phosphate; and the second component comprises: an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst; pentaerythritol tetrakis(3-mercaptopropionate); tributylamine; white petrolatum; and β-tricalcium phosphate. In certain aspects, the first component comprises: 35 to 45% (e.g., at least, at most, exactly, or between (inclusive or exclusive) of 5%, 36%, 37%, 38%, 39%, 40%, 41%, 42%, 43%, 44%, or 45%) by weight of an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst; 1 to 10% (e.g., at least, at most, exactly, or between (inclusive or exclusive) of 1%, 2%, 3%, 4%, 5%, 6%, 7%, 8%, 9%, or 10%) by weight of pentaerythritol tetrakis(3-mercaptopropionate); 0.00001 to 0.01% (e.g., at least, at most, exactly, or between (inclusive or exclusive) of 0.00001%, 0.00002%, 0.00003%, 0.00004%, 0.00005%, 0.00006%, 0.00007%, 0.00008%, 0.00009%, 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, or 0.01%) by weight of tributylamine; 0.1 to 5% (e.g., at least, at most, exactly, or between (inclusive or exclusive) of 0.1%, 0.2%, 0.3%, 0.4%, 0.5%, 0.6%, 0.7%, 0.8%, 0.9%, 1%, 2%, 3%, 4%, or 5%) by weight of white petrolatum; and 48 to 58% (e.g., at least, at most, exactly, or between (inclusive or exclusive) of 48%, 49%, 50%, 51%, 52%, 53%, 54%, 55%, 56%, 57%, or 58%) by weight of β-tricalcium phosphate; and the second component comprises: 2.5 to 12.5% (e.g., at least, at most, exactly, or between (inclusive or exclusive) of 2.5%, 3.5%, 4.5%, 5.5%, 6.5%, 7.5%, 8.5%, 9.5%, 10.5%, 11.5%, or 12.5%) by weight of an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst; 35 to 45% (e.g., at least, at most, exactly, or between (inclusive or exclusive) of 5%, 36%, 37%, 38%, 39%, 40%, 41%, 42%, 43%, 44%, or 45%) by weight of pentaerythritol tetrakis(3-mercaptopropionate); 0.0001 to 0.1% (e.g., at least, at most, exactly, or between (inclusive or exclusive) of 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, 0.01%, 0.02%, 0.03%, 0.04%, 0.05%, 0.06%, 0.07%, 0.08%, 0.09%, or 0.1%) by weight of tributylamine; 1 to 10% (e.g., at least, at most, exactly, or between (inclusive or exclusive) of 1%, 2%, 3%, 4%, 5%, 6%, 7%, 8%, 9%, or 10%) by weight of white petrolatum; and 45 to 55% (e.g., at least, at most, exactly, or between (inclusive or exclusive) of 45%, 46%, 47%, 48%, 49%, 50%, 51%, 52%, 53%, 54%, or 55%) by weight of β-tricalcium phosphate.

In certain aspects, the first component comprises: an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst; trimethylolpropane tris(3-mercaptopropionate); tributylamine; white petrolatum; and hydroxyapatite; and the second component comprises: isophorone diisocyanate; pentaerythritol tetrakis(3-mercaptopropionate); tributylamine; white petrolatum; and β-tricalcium phosphate. In certain aspects, the first component comprises: 30 to 40% (e.g., at least, at most, exactly, or between (inclusive or exclusive) of 30%, 31%, 32%, 33%, 34%, 35%, 36%, 37%, 38%, 39%, or 40%) by weight of an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst; 1 to 10% (e.g., at least, at most, exactly, or between (inclusive or exclusive) of 1%, 2%, 3%, 4%, 5%, 6%, 7%, 8%, 9%, or 10%) by weight of trimethylolpropane tris(3-mercaptopropionate); 0.00001 to 0.01% (e.g., at least, at most, exactly, or between (inclusive or exclusive) of 0.00001%, 0.00002%, 0.00003%, 0.00004%, 0.00005%, 0.00006%, 0.00007%, 0.00008%, 0.00009%, 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, or 0.01%) by weight of tributylamine; 0.1 to 5% (e.g., at least, at most, exactly, or between (inclusive or exclusive) of 0.1%, 0.2%, 0.3%, 0.4%, 0.5%, 0.6%, 0.7%, 0.8%, 0.9%, 1%, 2%, 3%, 4%, or 5%) by weight of white petrolatum; and 50 to 60% (e.g., at least, at most, exactly, or between (inclusive or exclusive) of 50%, 51%, 52%, 53%, 54%, 55%, 56%, 57%, 58%, 59%, or 60%) by weight of hydroxyapatite; and the second component comprises: 1 to 10% (e.g., at least, at most, exactly, or between (inclusive or exclusive) of 1%, 2%, 3%, 4%, 5%, 6%, 7%, 8%, 9%, or 10%) by weight of isophorone diisocyanate; 30 to 40% (e.g., at least, at most, exactly, or between (inclusive or exclusive) of 30%, 31%, 32%, 33%, 34%, 35%, 36%, 37%, 38%, 39%, or 40%) by weight of pentaerythritol tetrakis(3-mercaptopropionate); 0.00001 to 0.01% (e.g., at least, at most, exactly, or between (inclusive or exclusive) of 0.00001%, 0.00002%, 0.00003%, 0.00004%, 0.00005%, 0.00006%, 0.00007%, 0.00008%, 0.00009%, 0.0001%, 0.0002%, 0.0003%, 0.0004%, 0.0005%, 0.0006%, 0.0007%, 0.0008%, 0.0009%, 0.001%, 0.002%, 0.003%, 0.004%, 0.005%, 0.006%, 0.007%, 0.008%, 0.009%, or 0.01%) by weight of tributylamine; 1 to 10% (e.g., at least, at most, exactly, or between (inclusive or exclusive) of 1%, 2%, 3%, 4%, 5%, 6%, 7%, 8%, 9%, or 10%) by weight of white petrolatum; and 50 to 60% (e.g., at least, at most, exactly, or between (inclusive or exclusive) of 50%, 51%, 52%, 53%, 54%, 55%, 56%, 57%, 58%, 59%, or 60%) by weight of β-tricalcium phosphate.

In another aspect of the disclosure, disclosed is a bone repair composition for use in a method of repairing or reconstructing a bone defect in a subject, the method comprising: (a) mixing together a first component and a second component of any one of the bone repair compositions disclosed herein, thereby forming a polythiourethane; and (b) administering the resulting polythiourethane to the bone defect. In some aspects, the bone defect is a bone void, bone fracture, bleeding bone, or bone defect resulting from trauma, injury, infection, malignancy, and/or developmental malformation. In some aspects, the bone defect is a bone void, bone fracture, bleeding bone, or bone defect resulting from trauma, injury, infection, malignancy, and/or developmental malformation.

In another aspect of the disclosure, disclosed is a bone repair composition comprising a polythiourethane, wherein the polythiourethane is the reaction product of: (a) a first component comprising: (i) one or more isocyanates having at least two functional isocyanate groups, and/or (ii) one or more isocyanate-terminated prepolymers comprising one or more isocyanates having at least two functional isocyanate groups; and (b) a second component comprising: (i) one or more thiols having at least two functional thiol groups, and/or (ii) one or more thiol-based prepolymers comprising one or more thiols having at least two functional thiol groups, wherein upon mixing (a) and (b), the functional thiol groups of the second component react with the functional isocyanate groups of the first component, thereby forming the polythiourethane, wherein the polythiourethane cures into a solid form after the reaction is complete, and wherein the composition is biocompatible and/or bioresorbable in bone. In some aspects, the weight ratio of the first component to the second component is about 0.5:1 to about 5:1 (e.g., at least, at most, exactly, between (inclusive or exclusive), or about 0.5:1, 1:1, 1.5:1, 2:1, 2.5:1, 3:1, 3.5:1, 4:1, 4.5:1, or 5:1).

In another aspect of the disclosure, disclosed is a kit for bone repair comprising: (a) a first component comprising: (i) one or more isocyanates having at least two functional isocyanate groups, and/or (ii) one or more isocyanate-terminated prepolymers comprising one or more isocyanates having at least two functional isocyanate groups; (b) a second component comprising: (i) one or more thiols having at least two functional thiol groups, and/or (ii) one or more thiol-based prepolymers comprising one or more thiols having at least two functional thiol groups; (c) instructions for mixing (a) and (b) to produce a polythiourethane composition; and (d) instructions for administering the polythiourethane composition to bone and bone defects, wherein upon mixing (a) and (b), the functional thiol groups of the second component react with the functional isocyanate groups of the first component, thereby forming the polythiourethane composition, wherein the polythiourethane composition cures into a solid form after the reaction is complete, and wherein the polythiourethane composition is biocompatible and/or bioresorbable in bone. In some aspects, the first component and/or the second component are moldable putties. In some aspects, the polythiourethane composition is a moldable putty prior to curing. In some aspects, the first component (a) and second component (b) are individually sealed in pouches or containers. In some aspects, the sealed pouches or containers are vacuum sealed.

In another aspect of the disclosure, disclosed is a method of making a two-component bone repair composition, the method comprising: (a) providing a first component comprising: (i) one or more isocyanates having at least two functional isocyanate groups, and/or (ii) one or more isocyanate-terminated prepolymers comprising one or more isocyanates having at least two functional isocyanate groups; (b) providing a second component comprising: (i) one or more thiols having at least two functional thiol groups, and/or (ii) one or more thiol-based prepolymers comprising one or more thiols having at least two functional thiol groups; and (c) mixing (a) and (b) together to form a polythiourethane, wherein the functional thiol groups of the second component react with the functional isocyanate groups of the first component, thereby forming the polythiourethane, and wherein the resulting polythiourethane cures into a solid form after the reaction is complete. In some aspects, the first component and/or the second component are moldable putties. In some aspects, the polythiourethane composition is a moldable putty prior to curing. In some aspects, the weight ratio of the first component to the second component is about 0.5:1 to about 5:1 (e.g., at least, at most, exactly, between (inclusive or exclusive), or about 0.5:1, 1:1, 1.5:1, 2: 1, 2.5:1, 3:1, 3.5:1, 4:1, 4.5: 1, or 5:1). In some aspects, the first component and/or the second component further comprises a colorant. In some aspects, the first component comprises a first colorant, and wherein the second component does not comprise a colorant or comprises a second colorant that is different than the first colorant. In some aspects, the second component comprises a first colorant, and wherein the first component does not comprise a colorant or comprises a second colorant that is different than the first colorant. In some aspects, the resulting polythiourethane comprises a color that is different from a color of the first component and a color of the second component. In some aspects, the different color of the resulting polythiourethane is indicative that the first component and the second component have been mixed to homogeneity

The terms "room temperature" or "RT" as used herein mean a temperature of 20°-25° C.

The term "subject" as used herein means a vertebrate animal and includes mammals which includes human beings.

All percentages used herein are on a weight to weight (% w/w) basis unless otherwise specified.

The terms "optional" or "optionally" as used herein mean that the subsequently described event, circumstance, or material may or may not occur or be present, and that the description includes instances where the event, circumstance, or material occurs or is present and instances where it does not occur or is not present.

The terms "about" or "approximately" as used herein are defined as being close to as understood by one of skill in the art, and in one non-limiting aspect, the terms are defined to be within 10%, preferably within 5%, more preferably within 1%, and most preferably within 0.5%.

The term "substantially" and its variations are defined to include ranges within 10%, within 5%, within 1%, or within 0.5%.

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") as used herein are inclusive or open-ended and do not exclude additional, unrecited elements or method steps.

The use of the word "a" or "an" when used in conjunction with the terms "comprising", "having", "including", or "containing" (or any variations of these words) may mean "one," but it is also consistent with the meaning of "one or more," "at least one," and "one or more than one."

For purposes of this application, a number value with one or more decimal places can be rounded to the nearest whole number using standard rounding guidelines, i.e., round up if the number being rounded is 5, 6, 7, 8, or 9; and round down if the number being rounded is 0, 1, 2, 3, or 4. For example, 0.42 can be rounded to 0.4.

The compositions and methods for their use can "comprise," "consist essentially of," or "consist of" any of the ingredients or steps disclosed throughout the specification. With respect to the transitional phrase "consisting essentially of," in one non-limiting aspect, a basic and novel characteristic of the compositions and methods of the present disclosure is their utility for bone repair and reconstruction of bone defects such as bone voids and fractures.

It is contemplated that any aspect discussed in this specification can be implemented with respect to any method or composition of the disclosure, and *vice versa.* Furthermore, compositions of the disclosure can be used to achieve methods of the disclosure.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented below.
**FIG.** 1 shows two separate reactive components prior to mixing to form a polythiourethane, the first component comprising isocyanates and the second part comprising thiols. One of the first component or the second component is colored blue.
FIG. 2 shows a polythiourethane composition having a light blue color produced after mixing the first component and the second component of FIG. 1 to homogeneity.

### DETAILED DESCRIPTION

A discovery has been made that provides a solution to at least one or more of the problems associated with bone repair compositions. In one aspect, the solution can include the use of compositions comprising two separate reactive components that can be mixed together to form a polythiourethane, the first component comprising isocyanates and the second part comprising thiols. The first component and/or the second component can further comprise a colorant to provide a visual indicator that the first component and the second component have been mixed sufficient (e.g., to homogeneity). Mixing to homogeneity can include mixing the first and second components such as that the resulting composition has a relatively consistent color. The color of the resulting composition can be different in color than the first and/or second components. The compositions of the disclosure are characterized by unexpected curing times that are sufficient for administration of the polythiourethane before curing of the polythiourethane to a hard solid form. This allows for a sufficient amount of time to apply the compositions of the present disclosure to a subject and a relatively quick waiting time to ensure that the compositions are set in place/cured. The reduced waiting time can be beneficial to the subject being treated and/or person administering the compositions of the present disclosure, as it can reduce the overall time spent during the treatment procedure, which can reduce costs and/or medical issues that may arise during the treatment procedure.

These and other non-limiting aspects of the present invention are described in further detail below.

### I. COMPOSITIONS

The present disclosure discloses bone repair compositions that comprise at least two separate reactive components (parts) that when mixed together form a polythiourethane that cures to a hard solid. The first component comprises isocyanates having functional isocyanate groups and/or isocyanate-terminated prepolymers, and the second component comprises thiols having functional thiol groups and/or thiol-based prepolymers. The compositions can comprise multiple separate components of isocyanates and/or isocyanate-terminated prepolymers and multiple separate components of thiols and/or thiol-based prepolymers. When the first component comes in contact with or is mixed with the second component, the functional thiol groups react with the isocyanate groups, and a polythiourethane is formed as a product of the reaction. The resulting polythiourethane is suitable for use as a bone cement, bone adhesive, bone void filler, bone graft substitute, and/or bone hemostatic agent for the repair and/or reconstruction of bone defects in a subject in need thereof.

The first component and/or second component of the compositions can be in a liquid form or in the form of a moldable putty. In some aspects, the first component and the second component are in liquid form and can be mixed together to form the polythiourethane using a mixing instrument such as a stirring rod or spatula. In other aspects, the first component and the second component are in the form of moldable putties, which can be mixed together to form the polythiourethane by hand kneading. In one aspect, the polythiourethane that results from combination of the first component and the second component is also in the form of a putty for a period of time after initiation of the curing reaction. The term "putty" refers to a composition that is soft, moldable, preferably non-elastic, and cohesive. The term "moldable" with regard to a putty or putties means that the putty can be shaped by hand manipulation.

The compositions, i.e., the first component and/or the second component, can further include one or more additives, such as colorants or dyes, that impart a color to the component. In one aspect, each component comprises a different colorant or dye such that when the component parts are combined to form the polythiourethane composition, the different colors mix to form a new color, and the new color is indicative that the composition has been mixed to homogeneity, i.e., that a single homogenous polythiourethane composition has been formed. As one example, the first component can include a colorant or dye which gives the first component a red color, the second component does not include a colorant or dye, and a polythiourethane composition of a substantially pink color is formed from the combination of the first component and the second component to homogeneity. As another example, the first component can include a colorant or dye which gives the first component a red color, the second component can include a colorant or dye which gives the second component a blue color, and a polythiourethane composition of a substantially purple color is formed from the combination of the first component and the second component to homogeneity. Non-limiting examples of the colors of the first and/or second colorants can include red, blue, pink, yellow, brown, purple, black, white, green, orange, gray, etc.

The first component and the second component can be mixed together to homogeneity by mixing the first component and the second component for about 10 seconds to about 10 minutes, e.g., at least, at most, exactly, between (inclusive or exclusive) any two of, or about any two of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, or 600 seconds. In some aspects, the first component and the second component are mixed together for about 1 minute. In some aspects, the first component and the second component are mixed together for about 2 minutes. In some aspects, the first component and the second component are mixed together for about 3 minutes. In some aspects, the first component and the second component are mixed together for about 4 minutes. In some aspects, the first component and the second component are mixed together for about 5 minutes.

The polythiourethane can cure to a hard solid in about 10 seconds to about 10 minutes, e.g., at least, at most, exactly, between (inclusive or exclusive) any two of, or about any two of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, or 600 seconds. In some aspects, the polythiourethane cures to a hard solid in less than 10 minutes. In some aspects, the polythiourethane cures to a hard solid in 1 minute. In some aspects, the polythiourethane cures to a hard solid in 1.25 minutes. In some aspects, the polythiourethane cures to a hard solid in 1.5 minutes. In some aspects, the polythiourethane cures to a hard solid in 2.5 minutes. In some aspects, the polythiourethane cures to a hard solid in 4 minutes. In some aspects, the polythiourethane cures to a hard solid in 5 minutes.

The compositions, i.e., the first component, the second component, and/or the polythiourethane, can be biocompatible and suitable for use *in vivo.* The term "biocompatible" refers to materials that do not induce undesirable effects when administered or implanted *in vivo,* for example, an immune reaction and/or an inflammatory reaction, or other adverse reaction that is detrimental to the subject to whom the composition(s) are administered. In one aspect, the compositions, i.e., the first component, the second component, and/or the polythiourethane, are fully or partially bioresorbable. The terms "bioresorbable" and "biodegradable" are used interchangeably herein and refer to the ability of the compositions to degrade (partially or completely) under physiological conditions into non-toxic products that can be metabolized or excreted from the body within a period of time, generally several days and up to a year or about 18 to 24 months (e.g., 18, 19, 20, 21, 22, 23, or 24 months) or longer. In one aspect, the compositions, i.e., the first component, the second component, and/or the polythiourethane, are fully bioresorbable within about 12 months. Compositions may be considered non-bioresorbable if they remain stable *in vivo* for periods exceeding about ten years.

The compositions, i.e., the first component, the second component, and/or the polythiourethane, can contain no added water. The compositions, i.e., the first component, the second component, and/or the polythiourethane, can be anhydrous. In certain aspects where there is no added water, water may nevertheless be present in small amounts. For example, certain optional particulate materials as described herein, such as β-tricalcium phosphate, may comprise bound water. Formulating the compositions in an atmosphere that contains moisture may also result in the incorporation of water into the compositions. In other aspects, the compositions are prepared under essentially water-free conditions with anhydrous components such that the resulting compositions are essentially anhydrous.

The compositions, i.e., the first component, the second component, and/or the polythiourethane, can be sterile. The compositions, i.e., the first component, the second component, and/or the polythiourethane, can be sterilizable (i.e., the first and second components can be packaged and terminally sterilized).

Disclosed below are descriptions of the contents of the separate components (e.g., first component and second component) of the compositions.

### A. Isocyanates & Isocyanate-Terminated Prepolymers

The first component comprises isocyanate-terminated prepolymers.

Non-limiting examples of isocyanates include methyl-2,6-diisocyanato hexanoate (MLDI), ethyl-2,6-diisocyanato hexanoate (ELDI), isophorone diisocyanate (IPDI), toluene diisocyanate (TDI), 1,4-butane diisocyanate (BDI), 4,4'-methylene diphenyl diisocyanate (MDI), hexamethylene diisocyanate (HDI), 2,2,4-trimethyl hexamethylene diisocyanate (TMHDI), cyclohexyl methylene diisocyanate (H12MDI), methylene-bis(4-cyclohexylisocyanate), 4,4'-Methylenebis(cyclohexyl isocyanate) (MBCHI), 1,4-cyclohexane diisocyanate (CHDI), tetraethyl methylene diphenyl diisocyanate (TMDI), dianisidine diisocyanate (DADI), m-tetramethyl xylene diisocyanate (m-TMXDI), p-tetramethyl xylene diisocyanate (p-TMXDI), 1,8-diisocyanatooctane, 1,10-diisocyanatodecane, 1,12-diisocyanatododecane, lysine diisocyanate, lysine triisocyanate (LTI), poly(hexamethylene diisocyanate) (PHMDI), poly(propylene glycol) tolylene 2,4 diisocyanate (PPGTDI), or combinations thereof. These isocyanates are commercially available through suppliers such as Sigma-Aldrich.

Isocyanate-terminated prepolymers are the reaction product of an excess of one or more isocyanates having two or more isocyanate groups with a compound having one or more functional nucleophilic groups including, but not limited to polyols, polyamines, and thiols. The polyols can have at least two functional hydroxyl (-OH) groups. The thiols can have at least two thiol (-SH) groups. However, once reacted, the isocyanate-terminated prepolymers do not have functional hydroxyl groups present. Examples of suitable isocyanate-terminated prepolymers and methods of making such prepolymers can be found in U.S. Pat. No. 8,628,761. An exemplary isocyanate-terminated prepolymer manufactured by PolyNovo Biomaterials Pty Ltd is the reaction product of an excess of hexamethylene diisocyanate (HDI) and/or isophorone diisocyanate (IPDI) with pentaerythritol (a polyol) plus dibutyltin dilaurate as a catalyst.

The isocyanate-terminated prepolymer is the reaction product of an excess of isocyanate with a polyol can further be reacted in excess with a thiol, thereby forming a new isocyanate-terminated prepolymer. For example, an isocyanate-terminated prepolymer that is the reaction product of an excess of hexamethylene diisocyanate (HDI) and/or isophorone diisocyanate (IPDI) with pentaerythritol (a polyol) plus dibutyltin dilaurate as a catalyst can further be reacted in excess with a thiol such as pentaerythritol tetrakis(3-mercaptoproprionate), thereby forming a new isocyanate-terminated prepolymer.

The weight ratios of the excess isocyanate or isocyanate-terminated prepolymer to thiol in the first component can be from about 2:1 to about 15:1 or from about 5:1 to about 10:1. The weight ratio of the excess isocyanate or isocyanate-terminated prepolymer to thiol in the first component can be at least, at most, exactly, between (inclusive or exclusive) any two of, or about 2:1, 3:1, 4:1, 5:1, 6:1, 7:1, 8:1, 9:1, 10:1, 11:1, 12:1, 13:1, 14:1, or 15:1. The molar ratios of the -NCO:-SH in the first component can be from about 2:1 to about 15:1 or from about 5:1 to about 10:1, e.g., at least, at most, exactly, between (inclusive or exclusive) any two of, or about 2:1, 3:1, 4:1, 5:1, 6:1, 7:1, 8:1, 9:1, 10:1, 11:1, 12:1, 13:1, 14:1, or 15:1.

In some aspects, the first component comprises 30, 31, 32, 33, 34, 35, 36, 37, 38, 39, 40, 41, 42, 43, 44, or 45% w/w of the one or more isocyanate-terminated prepolymers based on the total amount of the first component.

The amount of the one or more isocyanate-terminated prepolymers in the first component can be present in the first component at 45% w/w of the total amount of the first component.

In some aspects, the first component is sterile or sterilizable. In some aspects, the first component does not include a polyol, polyamine, and/or acrylate. In some aspects, the first component is anhydrous. In some aspects, the first component does not include added water.

### B. Thiols & Thiol-Based Prepolymers

The first component comprises one or more thiols. Also the second component comprises two or more thiols. The thiols have two or more functional thiol groups. The thiols are nucleophilic and are reactive toward isocyanates.

Examples of thiols include methanethiol, ethanethiol, 1-propanethiol, 2-propanethiol, 1,3-propanedithiol, 2-propenethiol, butanethiol, *tert-*butyl mercaptan, pentanethiol, thiophenol, 1-hexadecanethiol, pentachlorobenzenethiol, pentaerythritol tetrakis(3-mercaptoproprionate) (PTMPP), trimethylolpropane tris(3-mercaptopropionate) (TMPMP), HS-PEG1500-SH, 4arm-PEG10K-SH, 4arm-PEG15K-SH, 4arm-PEG20K-SH, or combinations thereof. In some aspects, the thiols do not have any functional hydroxyl (-OH) groups and/or functional amino (-NH₂) groups). These thiols are commercially available through suppliers such a Sigma-Aldrich.

The second component can comprise or further comprise one or more thiol-based prepolymers. In some aspects, thiol-based prepolymers are the reaction product of an excess of one or more thiols having at least two functional thiol groups with one or more isocyanates having at least two functional isocyanate groups or are the reaction product of an excess of one or more thiols having at least two functional thiol groups with one or more isocyanate-terminated prepolymers. In some aspects, the thiol-based prepolymers are terminated with functional thiol groups and/or have branched chains ending with functional thiol groups. In some aspects, the thiol-based prepolymers do not have any functional hydroxyl (-OH) groups and/or functional amino (-NH₂) groups.

The weight ratios of the isocyanate or isocyanate-terminated prepolymer to excess thiol in the second component can be from about 1:2 to about 1:15, or from about 1:5 to about 1:10. The weight ratio of the isocyanate or isocyanate-terminated prepolymer to excess thiol in the second component can be at least, at most, exactly, or between (inclusive or exclusive) any two of, or about 1:2, 1:3, 1:4, 1:5, 1:6, 1:7, 1:8, 1:9, 1:10, 1:11, 1:12, 1:13, 1:14, or 1:15. The molar ratio of -NCO:-SH in the second component can be from about 1:2 to about 1:15, or from about 1:5 to about 1:10, e.g., at least, at most, exactly, or between (inclusive or exclusive) any two of, or about 1:2, 1:3, 1:4, 1:5, 1:6, 1:7, 1:8, 1:9, 1:10, 1:11, 1:12, 1:13, 1:14, or 1:15.

In some aspects, the second component comprises 30, 31, 32, 33, 34, 35, 36, 37, 38, 39, 40, 41, 42, 43, 44, or 45% w/w of the one or more thiols based on the total amount of the second component.

The amount of the one or more thiols in the second component is present in the second component at 30% to 40%, or 30% to 35%, or 35% to 45%, or 35% to 40%, or 40% to 45% w/w of the total amount of the second component.

In some aspects, the second component is sterile or sterilizable. In some aspects, the first component and/or the second component is sterile or sterilizable. In some aspects, the second component does not include a polyol, polyamine, and/or acrylate. In some aspects, the first component and/or the second component does not include a polyol, polyamine, and/or acrylate. In some aspects, the second component is anhydrous. In some aspects, the first component and/or the second component is anhydrous. In some aspects, the second component does not include added water. In some aspects, the first component and/or the second component does not include added water.

### C. Additional Ingredients

The compositions disclosed herein (i.e., the first component and/or the second component) can further comprise excipients, functional ingredients, and/or additional ingredients. Non-limiting examples of such ingredients include active pharmaceutical ingredients (APIs), absorbents, antimicrobial agents, antioxidants, antibiofilm agents, antibiotics, binders, buffering agents, bulking agents, carbonates, catalysts, chelating agents, colorants, dyes, biocides, deodorant agents, emulsion stabilizers, film formers, fragrance ingredients, growth factors, gellants (e.g., cellulose ethers, microcrystalline cellulose, acrylic polymers, alginates, gums, organoclays), hydrocarbons, inorganic mineral compounds, opacifying agents, oxidizing agents, pH adjusters, plasticizers, preservatives (e.g., methylparaben, propylparaben, benzyl alcohol), reducing agents, emollients, humectants, polyethylene glycols, moisturizers, surfactants, emulsifying agents, foaming agents, hydrotopes, solvents, suspending agents, rheology modifiers, viscosity control agents, vulnerary agents, propellants, porogens, and/or chain extenders.

It is contemplated that the compositions of the present disclosure can include any amount of the ingredients discussed in this specification. The compositions can also include any number of combinations of additional ingredients described throughout this specification. The concentrations of the any ingredient within the compositions can vary. In non-limiting aspects, for example, the compositions can comprise, consist essentially of, or consist of, in their final form, for example, at least, at most, exactly, between (inclusive or exclusive) any two of, or about 0.0001%, 0.0002%, 0.0003%, 0.0004%, 0.0005%, 0.0006%, 0.0007%, 0.0008%, 0.0009%, 0.0010%, 0.0011%, 0.0012%, 0.0013%, 0.0014%, 0.0015%, 0.0016%, 0.0017%, 0.0018%, 0.0019%, 0.0020%, 0.0021%, 0.0022%, 0.0023%, 0.0024%, 0.0025%, 0.0026%, 0.0027%, 0.0028%, 0.0029%, 0.0030%, 0.0031%, 0.0032%, 0.0033%, 0.0034%, 0.0035%, 0.0036%, 0.0037%, 0.0038%, 0.0039%, 0.0040%, 0.0041%, 0.0042%, 0.0043%, 0.0044%, 0.0045%, 0.0046%, 0.0047%, 0.0048%, 0.0049%, 0.0050%, 0.0051%, 0.0052%, 0.0053%, 0.0054%, 0.0055%, 0.0056%, 0.0057%, 0.0058%, 0.0059%, 0.0060%, 0.0061%, 0.0062%, 0.0063%, 0.0064%, 0.0065%, 0.0066%, 0.0067%, 0.0068%, 0.0069%, 0.0070%, 0.0071%, 0.0072%, 0.0073%, 0.0074%, 0.0075%, 0.0076%, 0.0077%, 0.0078%, 0.0079%, 0.0080%, 0.0081%, 0.0082%, 0.0083%, 0.0084%, 0.0085%, 0.0086%, 0.0087%, 0.0088%, 0.0089%, 0.0090%, 0.0091%, 0.0092%, 0.0093%, 0.0094%, 0.0095%, 0.0096%, 0.0097%, 0.0098%, 0.0099%, 0.0100%, 0.0200%, 0.0250%, 0.0275%, 0.0300%, 0.0325%, 0.0350%, 0.0375%, 0.0400%, 0.0425%, 0.0450%, 0.0475%, 0.0500%, 0.0525%, 0.0550%, 0.0575%, 0.0600%, 0.0625%, 0.0650%, 0.0675%, 0.0700%, 0.0725%, 0.0750%, 0.0775%, 0.0800%, 0.0825%, 0.0850%, 0.0875%, 0.0900%, 0.0925%, 0.0950%, 0.0975%, 0.1000%, 0.1250%, 0.1500%, 0.1750%, 0.2000%, 0.2250%, 0.2500%, 0.2750%, 0.3000%, 0.3250%, 0.3500%, 0.3750%, 0.4000%, 0.4250%, 0.4500%, 0.4750%, 0.5000%, 0.5250%, 0.0550%, 0.5750%, 0.6000%, 0.6250%, 0.6500%, 0.6750%, 0.7000%, 0.7250%, 0.7500%, 0.7750%, 0.8000%, 0.8250%, 0.8500%, 0.8750%, 0.9000%, 0.9250%, 0.9500%, 0.9750%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, 5.9%, 6.0%, 6.1%, 6.2%, 6.3%, 6.4%, 6.5%, 6.6%, 6.7%, 6.8%, 6.9%, 7.0%, 7.1%, 7.2%, 7.3%, 7.4%, 7.5%, 7.6%, 7.7%, 7.8%, 7.9%, 8.0%, 8.1%, 8.2%, 8.3%, 8.4%, 8.5%, 8.6%, 8.7%, 8.8%, 8.9%, 9.0%, 9.1%, 9.2%, 9.3%, 9.4%, 9.5%, 9.6%, 9.7%, 9.8%, 9.9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 60%, 65%, 70%, 75%, 80%, 85%, 90%, 95%, or 99%, of at least one of the ingredients that are mentioned throughout the specification and claims. In non-limiting aspects, the percentage can be calculated by weight or volume of the total composition. A person of ordinary skill in the art would understand that the concentrations can vary depending on the addition, substitution, and/or subtraction of ingredients in a given composition.

### a. Colorants

In some aspects, the first component and/or second component of the composition further comprises one or more colorants. In some aspects, the colorant is added during mixing of the first component and the second component, and the resulting polythiourethane composition becomes uniform in color when homogeneity of the first component and the second component is achieved. In some aspects, the colorant is a biocompatible dye. Non-limiting examples of biocompatible dyes include D&C Blue No. 6, D&C Blue No. 9, D&C Green No. 5, D&C Green No. 6, D&C Violet No. 2, D&C Black No. 4, FD&C Blue No. 2, chlorophyllin-copper complex, chromium-cobalt-aluminum oxide, [phthalocyaninato(2-)] copper, ferric ammonium citrate, pyrogallol, and/or logwood extract. In some aspects, the colorant is D&C Green No. 6. In some aspects, the colorant is D&C Violet No. 2. In some aspects, the colorant is D&C Blue No. 6.

Additionally, or alternatively, one or more ingredients of the first component and/or the second component may be colored. As one non-limiting example, in one aspect, the first and/or second component can include a colored inorganic mineral compound (e.g., colored hydroxyapatite). As another non-limiting example, the first or second component can include polymer microspheres having a color that is different from the color of the polymer or prepolymer ingredients of the other of the first and second components.

In one aspect, each of the first and second components comprise a different colorant or dye such that when the first and second components are combined to form the polythiourethane composition, the different colors mix to form a new color, and the new color is indicative that the composition has been mixed to homogeneity, i.e., that a single homogenous composition has been formed. As one example, the first component can include a colorant or dye which gives the first component a red color, the second component does not include a colorant or dye, and a composition of a substantially pink color is formed from the combination of the first component and the second component to homogeneity. As another example, the first component can include a colorant or dye which gives the first component a red color, the second component can include a colorant or dye which gives the second component a blue color, and a composition of a substantially purple color is formed from the combination of the first component and the second component to homogeneity. Inclusion of a colorant as a visual indicator that the first component and the second component have been mixed to homogeneity can, in some aspects, enhance safety of the first component, the second component, and/or the polythiourethane composition by reducing risk of undermixed components. The colorant may also increase the ease of use of the first component, the second component, and/or the polythiourethane composition as a bone repair composition. Non-limiting examples of the colors of the first and/or second colorants can include red, blue, pink, yellow, brown, purple, black, white, green, orange, gray, etc.

Concentrations of colorants can be from about 0.00001% to about 1% w/w, or about 0.0001% to about 0.5% w/w, or about 0.001% to about 0.1% w/w, or about 0.001% to about 0.01% w/w of the total amount of the first component, the total amount of the second component, the total amount of the first component and the total amount of the second component, or the total amount of the combination of the first component and the second component (e.g., at least, at most, exactly, between (inclusive or exclusive) any two of, or about 0.00001, 0.00002, 0.00003, 0.00004, 0.00005, 0.00006, 0.00007, 0.00008, 0.00009, 0.0001, 0.0002, 0.0003, 0.0004, 0.0005, 0.0006, 0.0007, 0.0008, 0.0009, 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, or 1% w/w of the total amount of the first component, the total amount of the second component, the total amount of the first component and the total amount of the second component, or the total amount of the combination of the first component and the second component.

### b. Catalysts

The first component and/or second component of the composition further comprises tributylamine (TBA) as catalyst.

Concentrations of catalysts can be from about 0.0001% to about 0.01% w/w of the total amount of the first component, or the total amount of the second component (e.g., at least, at most, exactly, between (inclusive or exclusive) any two of, or about 0.0001, 0.0002, 0.0003, 0.0004, 0.0005, 0.0006, 0.0007, 0.0008, 0.0009, 0.001, 0.002, 0.003, 0.004, 0.005, 0.006, 0.007, 0.008, 0.009, 0.01% w/w of the total amount of the first component, or the total amount of the second component).

### c. Inorganic Mineral Compounds

The first component and second component of the composition further comprises one or more inorganic mineral compounds, preferably particulate inorganic mineral compounds. Examples of particulate inorganic mineral compounds include hydroxyapatite or β-tricalcium phosphate. In some aspects, the particulate inorganic mineral compound is β-tricalcium phosphate.

The particles size of the particulate inorganic mineral compounds (e.g., β-tricalcium phosphate) can be at least, at most, exactly, between (inclusive or exclusive) any two of, or about 1, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1100, 1110, 1120, 1130, 1140, 1150, 1160, 1170, 1180, 1190, 1200, 1210, 1220, 1230, 1240, 1250, 1260, 1270, 1280, 1290, 1300, 1310, 1320, 1330, 1340, 1350, 1360, 1370, 1380, 1390, 1400, 1410, 1420, 1430, 1440, 1450, 1460, 1470, 1480, 1490, 1500, 1510, 1520, 1530, 1540, 1550, 1560, 1570, 1580, 1590, 1600, 1610, 1620, 1630, 1640, 1650, 1660, 1670, 1680, 1690, 1700, 1710, 1720, 1730, 1740, 1750, 1760, 1770, 1780, 1790, 1800, 1810, 1820, 1830, 1840, 1850, 1860, 1870, 1880, 1890, 1900, 1910, 1920, 1930, 1940, 1950, 1960, 1970, 1980, 1990, 2000, 2010, 2020, 2030, 2040, 2050, 2060, 2070, 2080, 2090, 2100, 2110, 2120, 2130, 2140, 2150, 2160, 2170, 2180, 2190, 2200, 2210, 2220, 2230, 2240, 2250, 2260, 2270, 2280, 2290, 2300, 2310, 2320, 2330, 2340, 2350, 2360, 2370, 2380, 2390, 2400, 2410, 2420, 2430, 2440, 2450, 2460, 2470, 2480, 2490, or 2500 microns in size.

The particles size of the particulate inorganic mineral compounds (e.g., β-tricalcium phosphate) can be from about 5 microns to about 2500 microns, or about 5 microns to about 2000 microns, or about 5 microns to about 1500 microns, or about 5 microns to about 1000 microns, or about 5 microns to about 750 microns, or about 5 microns to about 500 microns, or about 5 microns to about 400 microns, or about 5 microns to about 300 microns, or about 5 microns to about 250 microns, or about 5 microns to about 200 microns, or about 5 microns to about 150 microns, or about 5 microns to about 140 microns, or about 5 microns to about 130 microns, or about 5 microns to about 120 microns, or about 5 microns to about 110 microns, or about 5 microns to about 100 microns, or about 5 microns to about 90 microns, or about 5 microns to about 80 microns, or about 5 microns to about 70 microns, or about 5 microns to about 60 microns, or about 10 microns to about 2500 microns, or about 10 microns to about 2000 microns, or about 10 microns to about 1500 microns, or about 10 microns to about 1000 microns, or about 10 microns to about 750 microns, or about 10 microns to about 500 microns, or about 10 microns to about 400 microns, or about 10 microns to about 300 microns, or about 10 microns to about 250 microns, or about 10 microns to about 200 microns, or about 10 microns to about 150 microns, or about 10 microns to about 140 microns, or about 10 microns to about 130 microns, or about 10 microns to about 120 microns, or about 10 microns to about 110 microns, or about 10 microns to about 100 microns, or about 10 microns to about 90 microns, or about 10 microns to about 80 microns, or about 10 microns to about 70 microns, or about 10 microns to about 60 microns, or about 15 microns to about 2500 microns, or about 15 microns to about 2000 microns, or about 15 microns to about 1500 microns, or about 15 microns to about 1000 microns, or about 15 microns to about 750 microns, or about 15 microns to about 500 microns, or about 15 microns to about 400 microns, or about 15 microns to about 300 microns, or about 15 microns to about 250 microns, or about 15 microns to about 200 microns, or about 15 microns to about 150 microns, or about 15 microns to about 140 microns, or about 15 microns to about 130 microns, or about 15 microns to about 120 microns, or about 15 microns to about 110 microns, or about 15 microns to about 100 microns, or about 15 microns to about 90 microns, or about 15 microns to about 80 microns, or about 15 microns to about 70 microns, or about 15 microns to about 60 microns, or about 20 microns to about 2500 microns, or about 20 microns to about 2000 microns, or about 20 microns to about 1500 microns, or about 20 microns to about 1000 microns, or about 20 microns to about 750 microns, or about 20 microns to about 500 microns, or about 20 microns to about 400 microns, or about 20 microns to about 300 microns, or about 20 microns to about 250 microns, or about 20 microns to about 200 microns, or about 20 microns to about 150 microns, or about 20 microns to about 140 microns, or about 20 microns to about 130 microns, or about 20 microns to about 120 microns, or about 20 microns to about 110 microns, or about 20 microns to about 100 microns, or about 20 microns to about 90 microns, or about 20 microns to about 80 microns, or about 20 microns to about 70 microns, or about 20 microns to about 60 microns, or about 20 microns to about 50 microns.

Concentrations of the particulate mineral compound (e.g., β-tricalcium phosphate) can be from about 50% w/w to 60% w/w, 48% w/w to 58% w/w or 45% w/w to 55% w/w of the total amount of the first component, or the total amount of the second component (e.g., at least, at most, exactly, between (inclusive or exclusive) any two of, or about 45, 46, 47, 48, 49, 50, 51, 52, 53, 54, 55% w/w of the total amount of the first component, or the total amount of the second component).

The particles of the particulate mineral compound (e.g., β-tricalcium phosphate) used in the component parts of the compositions described here may be porous or nonporous. In one aspect, the particles are porous, and the degree of porosity is sufficient to permit the ingress of cells or fluids into the composition after its placement *in situ.*

### d. Plasticizers

The first component and/or second component of the composition further comprises one or more plasticizers. Examples of plasticizers are petrolatum, and white petrolatum.

Concentrations of the one or more plasticizers can be from 0.1% to 5% w/w, or from 1 % to 10% w/w of the total amount of the first component, or the total amount of the second component (e.g., at least, at most, exactly, between (inclusive or exclusive) any two of, or about 0.1, 0.2, 0.3, 0.4, 0.5, 0.6, 0.7, 0.8, 0.9, 1, 1, 2, 3, 4, 5, 6, 7, 8, 9, 10% w/w of the total amount of the first component, or the total amount of the second component).

### e. Hydrocarbons

In some aspects, the first component and/or the second component of the composition further comprises one or more hydrocarbons. Non-limiting examples of hydrocarbons include mineral oil, petrolatum, white petrolatum, yellow petrolatum, and/or paraffin.

Concentration of the hydrocarbons can be from about 0.1% to about 50% w/w, or about 0.1% to about 40% w/w, or about 0.1% to about 30% w/w, or about 0.1% to about 25% w/w, or about 0.1% to about 20% w/w, or about 0.1% to about 15% w/w, or about 0.4% to about 50% w/w, or about 0.4% to about 40% w/w, or about 0.4% to about 30% w/w, or about 0.4% to about 25% w/w, or about 0.4% to about 20% w/w, or about 0.4% to about 15% w/w, about 1% to about 50% w/w, or about 1% to about 40% w/w, or about 1% to about 30% w/w, or about 1% to about 25% w/w, or about 1% to about 20% w/w, or about 1% to about 15% w/w of the total amount of the first component, the total amount of the second component, the total amount of the first component and the total amount of the second component, or the total amount of the combination of the first component and the second component (e.g., at least, at most, exactly, between (inclusive or exclusive) any two of, or about 0.1, 0.2, 0.3, 0.4, 0.5, 0.6, 0.7, 0.8, 0.9, 1, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50% w/w of the total amount of the first component, the total amount of the second component, the total amount of the first component and the total amount of the second component, or the total amount of the combination of the first component and the second component).

### f. Other Ingredients

In some aspects, the first component and/or the second component of the composition further comprises one or more porogens, and/or one or more chain extenders, and/or one or more antibiotics, and/or one of more growth factors. In some aspects, the composition (e.g., the first component and/or the second component) does not include or contain an active pharmaceutical ingredient.

### D. Polythiourethane Compositions

The reaction product of the reaction of the functional thiol groups of the thiols in the second component upon mixing with the isocyanate groups of the isocyanates in the first component of the compositions disclosed *supra* is a polythiourethane composition that cures into a hard solid form. The polythiourethane can be in a liquid form or in the form of a moldable putty prior to it curing. The polythiourethane is suitable for use as a bone cement, bone adhesive, bone void filler, bone graft substitute, and/or bone hemostatic agent. Preferably, the polythiourethane is biocompatible, bioresorbable, and/or biodegradable in bone. The polythiourethane can be anhydrous. The polythiourethane can be sterile or sterilizable.

The weight ratios of the first component to the second component can be from about 0.5:1 to about 5:1, or from about 0.5:1 to about 4:1, or from about 0.5:1 to about 3:1, or from about 0.5:1 to about 2:1, or from about 0.5:1 to about 1.75:1, or from about 0.5:1 to about 1.5:1, or from about 0.75:1 to about 5:1, or from about 0.75:1 to about 4:1, or from about 0.75:1 to about 3:1, or from about 0.75:1 to about 2:1, or from about 0.75:1 to about 1.75:1, or from about 0.75:1 to about 1.5:1, or from about 1:1 to about 5:1, or from about 1:1 to about 4:1, or from about 1:1 to about 3:1, or from about 1:1 to about 2:1, or from about 1:1 to about 1.75:1, or from about 1:1 to about 1.5:1. In some aspects, the weight ratio of the first component to the second component is at least, at most, exactly, between (inclusive or exclusive) any two of, or about 0.5:1, 0.6:1, 0.7:1, 0.8:1, 0.9:1, 1:1, 1.1:1, 1.2:1, 1.3:1, 1.4:1, 1.5:1, 1.6:1, 1.7:1, 1.8:1, 1.9:1, 2:1, 2.5:1, 3:1, 3.5:1, 4:1, 4.5:1, or 5:1.

### II. METHODS OF MAKING THE COMPOSITIONS

The two-component (i.e., first component and second component) bone repair compositions disclosed *supra* can be made using mixing equipment and techniques known to one of skill in the art. In some aspects, the method of making a two-component bone repair composition comprises (a) providing a first component comprising: (i) one or more isocyanates having at least two functional isocyanate groups and/or (ii) one or more isocyanate-terminated prepolymers comprising one or more isocyanates having at least two functional isocyanate groups; (b) providing a second component comprising: (i) one or more thiols having at least two functional thiol groups, and/or (ii) one or more thiol-based prepolymers comprising one or more thiols having at least two functional thiol groups; and (c) mixing (a) and (b) together to form a polythiourethane. The first component can be made by combining the ingredients of the first component together and mixing until homogenous. Heat may be used as necessary. The second component can be made by combining the ingredients of the second component together and mixing until homogenous. Heat may be used as necessary. The first component and second component can be packaged separately into individual packages, containers, syringes, or pouches (such as aluminum pouches), optionally with vacuum sealing.

In an exemplary method, the first component is made by the following procedure: an isocyanate(s) and/or an isocyanate prepolymer is added to a container with a mixer. Then, a thiol(s) is added in an amount such that the amount of isocyanate and/or isocyanate prepolymer is in excess with respect to the amount of thiol. Then, the container is heated to about 75° C, and the isocyanate and/or isocyanate prepolymer and thiol are mixed at about 75° C until a clear and transparent solution is obtained. Then, a catalyst(s) is added, and the isocyanate and/or isocyanate prepolymer, thiol, and catalyst are mixed at 75° C for a period of time (e.g., at least, at most, exactly, or between (inclusive or exclusive) of 5, 10, 15, 20, 30, 35, 40, 45, 50, 55, or 60 minutes) until the reaction between the isocyanate and the thiol is complete. In some aspects, the viscosity of the solution increases as the reaction between the isocyanate and the thiol progresses. Then, petrolatum is added, and the solution is mixed at 75° C until uniform and an opaque solution is formed, after which, heating is discontinued. Then, a portion of a particulate inorganic mineral compound(s), such as tricalcium phosphate or hydroxyapatite, is added to the solution, and the solution is mixed until uniform and a flowable suspension is formed. Then, the remaining portion of the inorganic mineral compound is added to the flowable suspension, and the flowable suspension is mixed until a viscous semi-solid slurry is formed. Then, the viscous semi-solid slurry is cooled to about 40-50° C while mixing. Then, the mixture is packaged (e.g., into a container or pouch) and vacuum sealed.

In an exemplary method, the second component is made by the following procedure: an isocyanate(s) and/or an isocyanate prepolymer is added to a container with a mixer. Then, a thiol(s) is added in an amount such that the amount of thiol is in excess with respect to the amount of isocyanate and/or isocyanate prepolymer. Then, the container is heated to about 75° C, and the thiol and isocyanate and/or isocyanate prepolymer are mixed at about 75° C until a clear and transparent solution is obtained. Then, a catalyst(s) is added, and the thiol, isocyanate and/or isocyanate prepolymer, and catalyst are mixed at 75° C for a period of time (e.g., at least, at most, exactly, or between (inclusive or exclusive) of 5, 10, 15, 20, 30, 35, 40, 45, 50, 55, or 60 minutes) until the reaction between the isocyanate and the thiol is complete. In some aspects, the viscosity of the solution increases as the reaction between the isocyanate and the thiol progresses. Then, petrolatum is added, and the solution is mixed at 75° C until uniform and an opaque solution is formed, after which, heating is discontinued. Then, a portion of a particulate inorganic mineral compound(s), such as tricalcium phosphate or hydroxyapatite, is added to the solution, and the solution is mixed until uniform and a flowable suspension is formed. Then, the remaining portion of the inorganic mineral compound is added to the flowable suspension, and the flowable suspension is mixed until a viscous semi-solid slurry is formed. Then, the viscous semi-solid slurry is cooled to about 40-50° C while mixing. Then, the mixture is packaged (e.g., into a container or pouch) and vacuum sealed.

To make the polythiourethane, the first component and the second component of the compositions are mixed together. In some aspects, the first component and the second component of the compositions are mixed together for a period of time sufficient to produce a homogenous mixture. In some aspects, mixing the first component and the second component begins the reaction of forming the polythiourethane. In some aspects, the mixing time is from about 30 seconds to about 10 minutes, or from about 30 seconds to about 5 minutes, or from about 30 seconds to about 4 minutes, or from about 30 seconds to about 3 minutes, or from about 30 seconds to about 2 minutes, or from about 30 seconds to about 1 minutes, or from about 1 minute to about 10 minutes, or from about 1 minute to about 5 minutes, or from about 1 minute to about 4 minutes, or from about 1 minute to about 3 minutes, or from about 1 minute to about 2 minutes, or from about 2 minutes to about 10 minutes, or from about 2 minutes to about 5 minutes, or from about 2 minutes to about 4 minutes, or from about 2 minutes to about 3 minutes.

The polythiourethane comprising a homogenous mixture of the first component and the second component then cures into a solid form within a period of time. The period of time for curing can vary depending on the type of compounds present in the first component and the second component of the composition and can be adjusted accordingly. The curing time should not be too short where the user does not have enough time to administer the polythiourethane before is cures into a solid form. In some aspects, the curing time is from about 1 minute to about 10 minutes.

In some aspects, the first component and the second component are in liquid form and can be mixed together to form the polythiourethane using a mixing instrument such as a stirring rod or spatula. In other aspects, the first component and the second component are in the form of moldable putties, which can be mixed together to form the polythiourethane by hand kneading (e.g., pressing the two components together and then repeatedly folding and flattening the resulting mixture). In one aspect, the polythiourethane that results from combination of the first component and the second component is also in the form of a putty for a period of time after initiation of the curing reaction. The term "putty" refers to a composition that is soft, moldable, preferably non-elastic, and cohesive. The term "moldable" with regard to a putty or putties means that the putty can be shaped by hand manipulation.

### III. METHODS OF USE

The compositions disclosed herein are suitable for use as a bone cement, bone adhesive, bone void filler, bone graft substitute, and/or bone hemostatic agent for the repair and/or reconstruction of bone and bone defects in a subject. In some aspects, a bone repair composition for use in a method of repairing or reconstructing a bone defect in subject comprises mixing together the first component and second component of the composition to form a polythiourethane and administering the resulting polythiourethane to the bone defect. Non-limiting examples of bone defects include a bone void, bone fracture, bleeding bone, and bone defect resulting from trauma, injury, infection, malignancy, and/or developmental malformation. The polythiourethane in liquid form can be administered, for example, by syringe or spatula. The polythiourethane in a moldable putty form can be administered, for example, by hand or with a spatula.

### IV. KITS

Disclosed herein are kits useful for bone repair comprising the compositions disclosed herein. As a practical matter, during use of the compositions in bone repair, freshly made polythiourethane may be required at different points in time. If the polythiourethane is not mixed just before use, its moldability, uniformity, and adherence to the surfaces to which it is administered will be diminished. In this context, the adhesive nature of the polythiourethane is a function of its uncured state. In some aspects, the polythiourethane as it cures bonds to the tissue at the site of administration, for example bone tissue, and if the polythiourethane is compounded too early, it may set before it can be applied. In such a state, it will be insufficiently moldable, insufficiently adhesive, and unsuitable for use. The kits described herein provide a solution to this problem by providing a curable polythiourethane by mixing together the first component and the second component as needed during a bone repair procedure.

In some aspects, a kit for bone repair comprises (a) a first component comprising: (i) one or more isocyanates having at least two functional isocyanate groups, and/or (ii) one or more isocyanate-terminated prepolymers comprising one or more isocyanates having at least two functional isocyanate groups; (b) a second component comprising: (i) one or more thiols having at least two functional thiol groups, and/or (ii) a thio-terminated prepolymer comprising one or more thiols having at least two functional thiol groups; (c) instructions for mixing (a) and (b) to produce a polythiourethane composition; and (d) instructions for administering the polythiourethane composition to bone and bone defects.

In some aspects, the first component and/or the second component are liquid, and the polythiourethane composition is a liquid prior to curing. In some aspects, the first component and/or the second component are liquid, and the polythiourethane composition is a moldable putty prior to curing. In some aspects, the first component and/or the second component are moldable putties, and the polythiourethane composition is also a moldable putty prior to curing. In some aspects, the first component (a) and second component (b) are individually sealed in pouches or containers, optionally with vacuum sealing.

In one aspect, provided is a container comprising two or more compartments, each compartment containing an amount of the first component or the second component. Where the container comprises multiple sets of components, the compartments are adapted such that each set can be removed without disturbing the other sets in the package. If the components are sterile, the container is adapted such that each set of components can be aseptically removed without compromising the sterility of the remaining sets. The construction of the container allows for the removal of a single set of components from their respective compartments just before use. The components are removed from the container and mixed together (e.g., until homogenous) to form a single polythiourethane composition for administration. In some aspects, the polythiourethane composition thus formed will initially be in a moldable form. As the polythiourethane composition sets, it hardens into a solid form.

In certain aspects, a kit has a shelf life of 1-3 years. In certain aspects, the kit has a shelf life of at least, at most, exactly, or between (inclusive or exclusive) any two of 1 month, 2 months, 3 months, 4 months, 5 months, 6 months, 7 months, 8 months, 9 months, 10 months, 11 months, 12 months, 13 months, 14 months, 15 months, 16 months, 17 months 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, or 36 months.

### EXAMPLES

As part of the disclosure of the present invention, specific examples are included below. The examples are for illustrative purposes only and are not intended to limit the invention in any manner. Those of ordinary skill in the art will readily recognize a variety of noncritical parameters, which can be changed or modified to yield essentially the same results.

### EXAMPLE 1

### (Polythiourethane Compositions from Isocyanate-Terminated Prepolymer & Thiol)

Polythiourethane compositions were formed by mixing a two-part composition comprising an isocyanate-terminated prepolymer ("Part 1"; first component) and a thiol ("Part 2"; second component).

The components of various lots of Part 1 are shown in Table 1 below.

**Table 1**

| | **Lot 0055-0113M01** | **Lot 0055-0122M01** | **Lot 0055-0111M01** | **Lot 0055-0110M02** |
|---|---|---|---|---|
| **Component** | **% w/w** | **% w/w** | **% w/w** | **% w/w** |
| Polymer A (PolyNovo)^{a} | 32 | 28.5 | 42 | 33 |
| White Petrolatum | 6 | 5.5 | 8 | 6.22 |
| Beta TCP^{b} (95 µm) | 62 | - | - | - |
| Beta TCP (137 µm) | - | 66 | - | - |
| Beta TCP (23 µm) | - | - | 50 | - |
| Beta TCP (56 µm) | - | - | - | 60.73 |

| | | | | |
|---|---|---|---|---|
| ^{a}Polymer A is supplied by PolyNovo and is an isocyanate-terminated prepolymer that is the reaction product of hexamethylene diisocyanate (HDI), isophorone diisocyanate (IPDI), and pentaerythritol, and dibutyltin dilaurate as a catalyst. ^{b}Beta TCP = β-tricalcium phosphate | | | | |

Part 1 was prepared by heating the white petrolatum to above 45° C to melt. Then, Polymer A was added while mixing and mixed with the white petrolatum for at least 5 minutes until a homogeneous emulsion was obtained. Then, approximately 50% of the total amount of the Beta TCP (β-tricalcium phosphate) was added to the emulsion while mixing to create a slurry while maintaining a temperature above 45° C. Then, after removing from heat, the remaining portion of Beta TCP was added to the slurry while mixing, and the resulting solution was cooled while mixing to room temperature to form a moldable putty.

The components of various lots of Part 2 are shown in Table 2 below.

**Table 2**

| | **Lot 0055-0215M01** | **Lot 0055-0215M02** | **Lot 0055-0217M01** | **Lot 0055-0217M02** | **Lot 0055-0217M03** | **Lot 055-0218M01** |
|---|---|---|---|---|---|---|
| **Component** | **% w/w** | **% w/w** | **% w/w** | **% w/w** | **% w/w** | **% w/w** |
| PDT^{a} | 8.09 | - | - | - | - | - |
| PTMPP^{b} | - | - | 31.75 | 27.78 | 32.0 | 25.0 |
| MPE^{c} | - | 7.83 | - | - | - | - |
| PEG-6000 Distearate | 0.918 | 1.03 | - | - | - | - |
| Triacetin | 31.5 | 30.0 | - | - | - | - |
| TEA^{d} | 0.405 | 0.391 | 0.059 | 0.0201 | 0.0115 | - |
| TBA^{e} | - | - | - | - | - | 0.00471 |
| SiO₂^{f} | 1.51 | 1.44 | - | - | - | - |
| Beta-TCP^{g} (23 µm) | 57.57 | 59.31 | 55.63 | 59.93 | 53.0 | 63.0 |
| White Petrolatum | - | - | 12.63 | 12.33 | 15.0 | 12.0 |

| | | | | | | |
|---|---|---|---|---|---|---|
| ^{a}PDT = 1,3 propanedithiol ^{b}PTMPP = pentaerythritol tetrakis(3-mercaptopropionate) ^{c}MPE = mercaptoethanol ^{d}TEA = triethylamine ^{e}TBA = tributylamine ^{f}SiO₂ = silicon dioxide ^{g}Beta TCP = β-tricalcium phosphate | | | | | | |

Part 2 was prepared by mixing the thiol component (PDT or PTMPP or MPE) with PEG-6000 distearate, and/or triacetin, and/or white petrolatum plus TEA or TBA with heating to 70° C until homogenous. After removing from heat, the Beta-TCP and the SiO₂, if present, were added to the solution, which was mixed until homogenous with cooling to RT to form a moldable putty.

Various polythiourethane compositions in the form of moldable putties were formed upon hand mixing (kneading) Part 1 and Part 2 *supra* together. Details of the polythiourethane compositions are shown in Table 3 below. The moldable putty polythiourethane compositions cured into hard solids by the end of the listed setting time.

**Table 3**

| **Polythiourethane Composition** | **Lot, Part 1** | **Lot, Part 2** | **Weight ratio Part 1:Part 2** | **Mixing time (sec)** | **Setting time (sec)** |
|---|---|---|---|---|---|
| A | 0055-0113M01 | 0055-0217M03 | 3:1 | 60 | 600 |
| B | 0055-0113M01 | 0055-0217M03 | 2:1 | 60 | 240 |
| C | 0055-0113M01 | 0055-0217M03 | 1:1 | 60 | 90 |
| D | 0055-0113M01 | 0055-0218M01 | 1.5:1 | 60 | 300 |
| E | 0055-0113M01 | 0055-0217MO2 | 1:1 | 60 | 60 |
| F | 0055-0113M01 | 0055-0217M02 | 2:1 | 60 | 75 |
| G | 0055-0113M01 | 0055-0217M01 | 3:1 | 60 | 150 |
| H | 0055-0111M01 | 0055-0215M01 | 1:1 | 60 | 60 |
| I | 0055-0113M01 | 0055-0215MO2 | 1:1 | 60 | >600 |

### EXAMPLE 2

### (Polythiourethane Compositions from Isocyanate-Terminated & Thiol-Based Prepolymers)

Polythiourethane compositions were formed by mixing a two-part composition comprising an isocyanate-terminated prepolymer ("Part 1"; first component) and a thiol-based prepolymer ("Part 2"; second component).

The components of various lots of Part 1 are shown in Table 4 below. The resulting free -NCO (isocyanate) is also shown for each lot.

**Table 4**

| | **Lot 0055-0329M01** | **Lot 0055-0406M01** | **Lot 0055-0406M02** |
|---|---|---|---|
| **Component** | **% w/w** | **% w/w** | **% w/w** |
| Polymer A (PolyNovo)^{a} | 40.8 | 39.61 | 40.21 |
| White Petrolatum | 1.34 | 1.66 | - |
| Beta TCP (58 µm)^{b} | 54.1 | 52.24 | 53.43 |
| PTMPP^{c} | 3.99 | 4.47 | 4.68 |
| TBA^{d} | 0.000547 | 0.000665 | 0.000726 |
| PVP^{e} | - | - | 1.68 |
| Free -NCO (mM) | 2.5 | 2.384 | 2.406 |

| | | | |
|---|---|---|---|
| ^{a}Polymer A is supplied by PolyNovo and is an isocyanate-terminated prepolymer that is the reaction product of hexamethylene diisocyanate (HDI), isophorone diisocyanate (IPDI), and pentaerythritol, and dibutyltin dilaurate as a catalyst. ^{b}Beta TCP = β-tricalcium phosphate ^{c}PTMPP = pentaerythritol tetrakis(3-mercaptopropionate) ^{d}TBA = tributylamine ^{e}PVP = polyvinylpyrrolidone | | | |

Part 1 was prepared by heating and mixing PTMPP and Polymer A to 70° C until a clear solution was obtained. Then, the TBA was added, and the mixture was stirred at 70° C for a period of time (e.g., at least, at most, exactly, or between (inclusive or exclusive) of 5, 10, 15, 20, 30, 35, 40, 45, 50, 55, or 60 minutes) until the reaction between the isocyanate and the thiol is complete. White petrolatum, PVP, if present, and approximately 50% of the total amount of the Beta TCP was added to the solution while mixing to create a slurry at 70° C. After removing from heat, the remaining amount of Beta TCP was added to the slurry while mixing, and the slurry was cooled to RT while mixing to form a moldable putty.

The components of various lots of Part 2 are shown in Table 5 below. The resulting free -SH (thiol) is also shown for each lot.

**Table 5**

| | **Lot 0055-0401M02** | **Lot 0055-0411M02** | **Lot 0055-0412M02** |
|---|---|---|---|
| **Component** | **% w/w** | **% w/w** | **% w/w** |
| Polymer A (PolyNovo)^{a} | 7.0 | 6.95 | 6.94 |
| White Petrolatum | 4.5 | 2.27 | 1.026 |
| Beta TCP (58 µm)^{b} | 49.53 | 49.17 | 49.21 |
| PTMPP^{c} | 39.38 | 39.38 | 39.36 |
| TBA^{d} | 0.001027 | 0.00144 | 0.001476 |
| PVP^{e} | - | 2.22 | 3.46 |
| Free -SH (mM) | 2.78 | 2.79 | 2.79 |

| | | | |
|---|---|---|---|
| ^{a}Polymer A is supplied by PolyNovo and is an isocyanate-terminated prepolymer that is the reaction product of hexamethylene diisocyanate (HDI), isophorone diisocyanate (IPDI), and pentaerythritol, and dibutyltin dilaurate as a catalyst. ^{b}Beta TCP = β-tricalcium phosphate ^{c}PTMPP = pentaerythritol tetrakis(3-mercaptopropionate) ^{d}TBA = tributylamine ^{e}PVP = polyvinylpyrrolidone | | | |

Part 2 was prepared by heating PTMPP and Polymer A to 50° to 70° C until a clear solution was obtained. Then, the TBA, white petrolatum, PVP, if present, and approximately 50% of the total amount of the Beta TCP was added to the solution while mixing to create a slurry. After removing from heat, the remaining amount of Beta TCP was added to the slurry while mixing and cooled to RT while mixing to form a moldable putty.

Various polythiourethane compositions in the form of moldable putties were formed upon hand mixing (kneading) Part 1 and Part 2 *supra* together. Details of the polythiourethane compositions are shown in Table 6 below. The moldable putty polythiourethane compositions cured into hard solids within the listed setting time.

**Table 6**

| **Polythiourethane Composition** | **Lot, Part 1** | **Lot, Part 2** | **Weight ratio Part 1:Part 2** | **Mixing time (min)** | **Setting time (min)** |
|---|---|---|---|---|---|
| J | 0055-0406M01 | 0055-0401M02 | 1:1 | 2.5-4 | <10 |
| K | 0055-0406M02 | 0055-0412M02 | 1.5:1 | 2.5-4 | <10 |
| L | 0055-0329M01 | 0055-0411M02 | 1:1 | 2.5-4 | <10 |

The calculated amounts of the components in the resulting polythiourethane compositions are shown in Table 7 below.

**Table 7**

| | **Polythiourethane Composition J** | **Polythiourethane Composition K** | **Polythiourethane Composition L** |
|---|---|---|---|
| **Component** | **% w/w** | **% w/w** | **% w/w** |
| Polymer A (PolyNovo)^{a} | 23.3 | 26.9 | 23.88 |
| PTMPP^{b} | 21.92 | 18.55 | 21.69 |
| Beta TCP^{c} (58 µm) | 51.7 | 51.75 | 52.15 |
| White Petrolatum | 3.08 | 0.41 | 1.14 |
| PVP^{d} | - | 2.39 | 1.11 |
| TBA^{e} | 0.000846 | 0.001024 | 0.000994 |

| | | | |
|---|---|---|---|
| "Polymer A is supplied by PolyNovo and is an isocyanate-terminated prepolymer that is the reaction product of hexamethylene diisocyanate (HDI), isophorone diisocyanate (IPDI), and pentaerythritol, and dibutyltin dilaurate as a catalyst. ^{b}PTMPP = pentaerythritol tetrakis(3-mercaptopropionate) ^{c}Beta TCP = β-tricalcium phosphate ^{d}PVP = polyvinylpyrrolidone ^{e}TBA = tributylamine | | | |

### EXAMPLE 3

### (Polythiourethane Compositions from Isocyanate-Terminated & Thiol-Based

### Prepolymers)

Polythiourethane compositions were formed by mixing a two-part composition comprising an isocyanate-terminated prepolymer ("Part 1"; first component) and a thiol-based prepolymer ("Part 2"; second component).

The components of various lots of Part 1 are shown in Table 8 below.

**Table 8**

| **Part 1** | **Lot 0055-0425M01** | **Lot 0055-0531M01** | **Lot 0055-0602M01** | **Lot 0055-0606M01** |
|---|---|---|---|---|
| **Component** | **% w/w** | **% w/w** | **% w/w** | **% w/w** |
| Polymer A (PolyNovo)^{a} | 39.6 | 23 | 36.82 | 29.6 |
| PTMPP^{b} | 4.48 | - | - | - |
| TMPMP^{c} | - | 3.83 | 6.19 | 4.04 |
| TBA^{d} | 0.000692 | 0.000538 | 0.000851 | 0.000585 |
| White Petrolatum | 1.68 | 1.17 | 1.96 | 1.22 |
| Beta TCP^{e} (58 µm) | 54.24 | - | - | - |
| Hydroxyapatite (26 µm) | - | 72 | 55.04 | 65.15 |

| | | | | |
|---|---|---|---|---|
| ^{a}Polymer A is supplied by PolyNovo and is an isocyanate-terminated prepolymer that is the reaction product of hexamethylene diisocyanate (HDI), isophorone diisocyanate (IPDI), and pentaerythritol, and dibutyltin dilaurate as a catalyst. ^{b}PTMPP = pentaerythritol tetrakis(3-mercaptopropionate) ^{c}TMPMP = trimethylolpropane tris(3-mercaptopropionate) ^{d}TBA = tributylamine ^{e}Beta TCP = β-tricalcium phosphate | | | | |

Part 1 was prepared by the following procedure: Polymer A was added to a container with a mixer. Then, the thiol (PTMPP or TMPMP) was added. Then, the container was heated to about 75° C, and the Polymer A and thiol were mixed at about 75° C until a clear and transparent solution was obtained. Then, the TBA was added, and the solution was mixed at 75° C for a period of time (e.g., at least, at most, exactly, or between (inclusive or exclusive) of 5, 10, 15, 20, 30, 35, 40, 45, 50, 55, or 60 minutes) until the reaction between the isocyanate and the thiol is complete (i.e., the viscosity of the solution increased). Then, the white petrolatum was added, and the solution was mixed at 75° C until uniform and an opaque solution was formed, and heating was discontinued. Then, a portion of the particulate inorganic mineral compound (tricalcium phosphate or hydroxyapatite) was added to the solution, and the solution was mixed until uniform and a flowable suspension was formed. Then, the remaining portion of the inorganic mineral compound was added to the flowable suspension, and the flowable suspension was mixed until a viscous semi-solid slurry was formed. Then, the viscous semi-solid slurry was cooled to about 40-50° C while mixing. The cooled slurry was then placed in an aluminum pouch and vacuum sealed.

The components of various lots of Part 2 are shown in Tables 9 and 10 below.

**Table 9**

| **Part 2** | **Lot 0055-0425M02** | **Lot 0055-0425M03** | **Lot 0055-0502M01** | **Lot 0055-0512M01** |
|---|---|---|---|---|
| **Component** | **% w/w** | **% w/w** | **% w/w** | **% w/w** |
| MBCHI^{a} | 5.43 | - | - | - |
| PHMDI^{b} | - | 5.86 | - | - |
| PPGTDI^{c} | - | - | 5.04 | - |
| IPDI^{d} | - | - | - | 5.75 |
| PTMPP^{e} | 30.21 | 32.63 | 27.77 | 35.52 |
| TMPMP^{f} | - | - | - | - |
| TBA^{g} | 0.000791 | 0.000878 | 0.000742 | 0.00093 |
| White Petrolatum | 3.48 | 3.79 | 3.32 | 4.28 |
| Beta TCP^{h} (58 µm) | 60.88 | 57.71 | 63.5 | 54.44 |
| Hydroxyapatite (26 µm) | - | - | - | - |

| | | | | |
|---|---|---|---|---|
| ^{a}MBCHI = 4,4'-Methylenebis(cyclohexyl isocyanate) ^{b}PHMDI = poly(hexamethylene diisocyanate) ^{c}PPGTDI = poly(propylene glycol) tolylene 2,4 diisocyanate ^{d}IPDI = isophorone diisocyanate ^{e}PTMPP = pentaerythritol tetrakis(3-mercaptopropionate) ^{f}TMPMP = trimethylolpropane tris(3-mercaptopropionate) ^{g}TBA = tributylamine ^{h}Beta TCP = β-tricalcium phosphate | | | | |

**Table 10**

| **Part 2** | **Lot 0055-0517M01** | **Lot 0055-0525M01** | **Lot 0055-0527M01** | **Lot 0055-0607M01** |
|---|---|---|---|---|
| **Component** | **% w/w** | **% w/w** | **% w/w** | **% w/w** |
| MBCHI^{a} | - | - | - | - |
| PHMDI^{b} | - | - | - | - |
| PPGTDI^{c} | - | - | - | - |
| IPDI^{d} | 7.05 | 5.47 | 5.16 | 4.79 |
| PTMPP^{e} | - | - | - | - |
| TMPMP^{f} | 33.02 | 19.44 | 21.56 | 26.2 |
| TBA^{g} | 0.000796 | 0.000542 | 0.000539 | 0.000789 |
| White Petrolatum | 4 | 2.06 | 2.23 | 3.21 |
| Beta TCP^{h} (58 µm) | 55.74 | - | - | - |
| Hydroxyapatite (26 µm) | - | 72.8 | 71.04 | 65.8 |

| | | | | |
|---|---|---|---|---|
| ^{a}MBCHI = 4,4'-Methylenebis(cyclohexyl isocyanate) ^{b}PHMDI = poly(hexamethylene diisocyanate) ^{c}PPGTDI = poly(propylene glycol) tolylene 2,4 diisocyanate ^{d}IPDI = isophorone diisocyanate ^{e}PTMPP = pentaerythritol tetrakis(3-mercaptopropionate) ^{f}TMPMP = trimethylolpropane tris(3-mercaptopropionate) ^{g}TBA = tributylamine ^{h}Beta TCP = β-tricalcium phosphate | | | | |

Part 2 was prepared by the following procedure: the isocyanate (MBCHI, PHMDI, PPGTDI, or IPDI) was added to a container with a mixer. Then, a thiol (PTMPP or TMPMP) was added. Then, the container was heated to about 75° C, and the isocyanate and thiol were mixed at about 75° C until a clear and transparent solution was obtained. Then, the TBA was added, and the solution was mixed at 75° C for a period of time (e.g., at least, at most, exactly, or between (inclusive or exclusive) of 5, 10, 15, 20, 30, 35, 40, 45, 50, 55, or 60 minutes) until the reaction between the isocyanate and the thiol is complete (i.e., the viscosity of the solution increased). Then, white petrolatum was added and mixed at 75° C until uniform and an opaque solution was formed, and heating was discontinued. Then, a portion of a particulate inorganic mineral compound (tricalcium phosphate or hydroxyapatite was added, and the solution was mixed until uniform and a flowable suspension was formed. Then, the remaining portion of the inorganic mineral compound was added to the flowable suspension, and the flowable suspension was mixed until a viscous semi-solid slurry was formed. Then, the viscous semi-solid slurry was cooled to about 40-50° C while mixing. The slurry was then placed in an aluminum pouch and vacuum sealed.

Various polythiourethane compositions in the form of moldable putties were formed upon hand mixing (kneading) Part 1 and Part 2 *supra* together. Details of the polythiourethane compositions are shown in Table 11 below. The moldable putty polythiourethane compositions cured into hard solids within the listed setting time.

**Table 11**

| **Polythiourethane Composition** | **Lot, Part 1** | **Lot, Part 2** | **Weight ratio Part 1:Part 2** | **Mixing time (min)** | **Setting time (min)** |
|---|---|---|---|---|---|
| M | 0055-0425M01 | 0055-0425M02 | 1.14:1 | 1-5 | <10 |
| N | 0055-0425M01 | 0055-0512M01 | 0.99:1 | 1-5 | <10 |
| O | 0055-0425M01 | 0055-0425M03 | 1.07:1 | 1-5 | <10 |
| P | 0055-0425M01 | 0055-0502M01 | 1.22:1 | 1-5 | <10 |
| Q | 0055-0602M01 | 0055-0512M01 | 0.88:1 | 1-5 | <10 |
| R | 0055-0602M01 | 0055-0525M01 | 1.42:1 | 1-5 | <10 |
| S | 0055-0602M01 | 0055-0527M01 | 1.33:1 | 1-5 | <10 |

### EXAMPLE 4

### (Colored Isocyanate-Terminated Prepolymer or Thiol Components)

Polythiourethane compositions are formed by mixing a two-part composition comprising an isocyanate-terminated prepolymer ("Part 1"; first component) and a thiol or thiol-based ("Part 2"; second component), as described above.

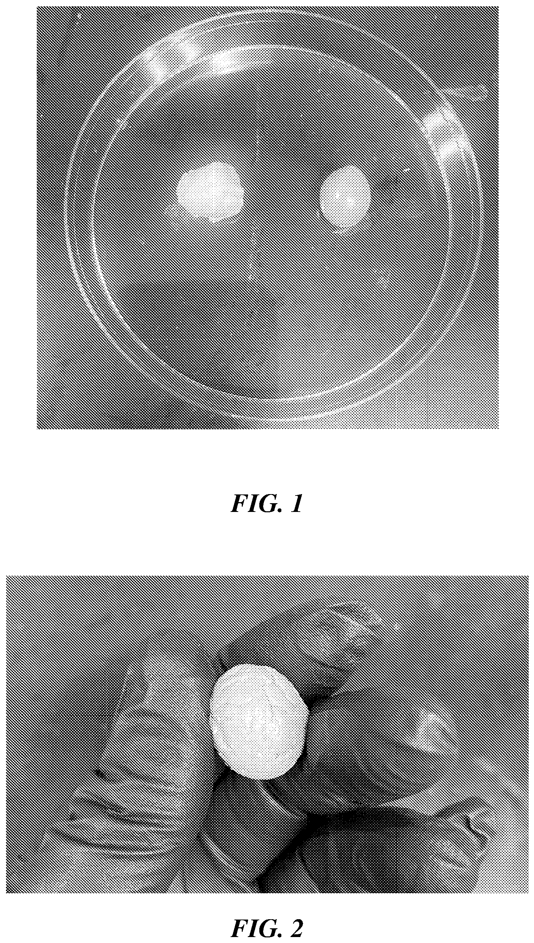

A colorant, or two different colorants, are added during the mixing process to Part 1, Part 2, or Part 1 and Part 2. For example, as shown in **FIG. 1****,** a blue colorant (e.g., D&C Blue No. 6) was added to either Part 1 or Part 2. Incorporating a colorant, or two different colorants, into Part 1 and/or Part 2 enables a visual change as the Parts are mixed. The color of the polythiourethane composition gradually changes as the Parts are mixed, until the polythiourethane composition comprises a color that is different from a color of Part 1 and a color of Part 2. For example, as shown in **FIG. 2****,** the polythiourethane composition produced comprises a lighter blue color that is different from a color of Part 1 and a color of Part 2. The different color of the polythiourethane composition is indicative that Part 1 and Part 2 have been mixed to homogeneity.

An exemplary formulation for Part 1 including a colorant is shown in Table 12 below.

**Table 12**

| **Component** | **% w/w** |
|---|---|
| Polymer A (PolyNovo)^{a} | 34.71 |
| TMPMP^{b} | 3.63 |
| Tributylamine | 0.00121 |
| White Petrolatum | 1.97 |
| Beta TCP^{c} (91 µm) | 59.66 |
| D&C Green No. 6 | 0.00152 |

| | |
|---|---|
| ^{a}Polymer A is supplied by PolyNovo and is an isocyanate-terminated prepolymer that is the reaction product of hexamethylene diisocyanate (HDI), isophorone diisocyanate (IPDI), and pentaerythritol, and dibutyltin dilaurate as a catalyst. ^{b}TMPMP = trimethylolpropane tris(3-mercaptopropionate) ^{c}Beta TCP = β-tricalcium phosphate | |

An exemplary formulation for Part 2 including a colorant is shown in Table 13 below.

**Table 13**

| **Component** | **% w/w** |
|---|---|
| TMPMP^{a} | 27.37 |
| IPDI | 4.33 |
| Tributylamine | 0.0.001207 |
| White Petrolatum | 3.25 |
| Beta TCP^{c} (53 µm) | 65.05 |
| D&C Green No. 6 | 0.0127 |

| | |
|---|---|
| ^{a}TMPMP = trimethylolpropane tris(3-mercaptopropionate) ^{b}IPDI = isophorone diisocyanate ^{c}Beta TCP = β-tricalcium phosphate | |

## Claims

1. A bone repair composition comprising two separate reactive components which can be mixed together to form a polythiourethane, wherein:
i) the first component comprises:
35 to 45% by weight of an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst;
1 to 10% by weight of pentaerythritol tetrakis(3-mercaptopropionate);
0.00001 to 0.01% by weight of tributylamine;
0.1 to 5% by weight of white petrolatum; and
50 to 60% by weight of β-tricalcium phosphate; and
the second component comprises:
1 to 10% by weight of isophorone diisocyanate;
30 to 40% by weight of pentaerythritol tetrakis(3 -mercaptopropionate);
0.00001 to 0.01% by weight of tributylamine;
1 to 10% by weight of white petrolatum; and
50 to 60% by weight of β-tricalcium phosphate;
ii) the first component comprises:
35 to 45% by weight of an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst;
1 to 10% by weight of pentaerythritol tetrakis(3-mercaptopropionate);
0.00001 to 0.01% by weight of tributylamine;
0.1 to 5% by weight of white petrolatum; and
48 to 58% by weight of β-tricalcium phosphate; and
the second component comprises:
2.5 to 12.5% by weight of an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst;
35 to 45% by weight of pentaerythritol tetrakis(3 -mercaptopropionate);
0.0001 to 0.1% by weight of tributylamine;
1 to 10% by weight of white petrolatum; and
45 to 55% by weight of β-tricalcium phosphate; or
iii) the first component comprises:
30 to 40% by weight of an isocyanate-terminated prepolymer comprising the reaction product of an excess of hexamethylene diisocyanate (HDI) and/or isophorone diisocyanate (IPDI) with pentaerythritol (a polyol) using dibutyltin dilaurate as a catalyst;
1 to 10% by weight of trimethylolpropane tris(3 -mercaptopropionate);
0.00001 to 0.01% by weight of tributylamine;
0.1 to 5% by weight of white petrolatum; and
50 to 60% by weight of hydroxyapatite; and
the second component comprises:
1 to 10% by weight of isophorone diisocyanate;
30 to 40% by weight of pentaerythritol tetrakis(3 -mercaptopropionate);
0.00001 to 0.01% by weight of tributylamine;
1 to 10% by weight of white petrolatum; and
50 to 60% by weight of β-tricalcium phosphate.

2. The bone repair composition of claim 1, wherein the first component and/or the second component are moldable putties, preferably wherein upon mixing of the first component and the second component, the resulting polythiourethane is a moldable putty prior to curing, preferably wherein the resulting polythiourethane is suitable for use as a bone cement, bone adhesive, bone void filler, bone graft substitute, and/or bone hemostatic agent.

3. The bone repair composition of any one of claims 1 to 2, wherein the first component and/or second component further comprises one or more catalysts,
preferably wherein the one or more catalysts comprise triethylamine, tributylamine, l,8-Diazabicyclo[5.4.0]undec-7-ene, dibutyltin dilaurate, stannous octoate, 1,4-diazabicyclo[2.2.2] octane, tetra n-butyl titanate, titanium acetylacetonate, triethanolamine titanate, titanium ethylaceto-acetate, tetraethyl titanate, tetraisopropyl titanate, titanium lactic acid chelate, triethylene diamine, bis(dimethylaminoethyl)ether, dimethylethanolamine, or combinations thereof.

4. The bone repair composition of any one of claims 1 to 3, wherein the first component and/or the second component further comprises one or more particulate inorganic mineral compounds,
preferably wherein the one or more particulate inorganic mineral compounds comprise aragonite, dahlite, calcite, calcium carbonate, amorphous calcium carbonate, vaterite, weddellite, whewellite, struvite, urate, ferrihydrite, francolite, monohydrocalcite, magnetite, goethite, dentin, calcium carbonate, calcium sulfate, calcium phosphosilicate, sodium phosphate, calcium aluminate, calcium phosphate, hydroxyapatite, a-tricalcium phosphate, P-tricalcium phosphate, dicalcium phosphate, 3-tricalcium phosphate, tetracalcium phosphate, amorphous calcium phosphate, octacalcium phosphate, calcium phosphate silica glass, fluoroapatite, chloroapatite, magnesium-substituted tricalcium phosphate, magnesium oxide, magnesium potassium phosphate, silica, silicon dioxide, soluble glass, sodium silicate, carbonate hydroxyapatite, or combinations thereof,
preferably wherein the particulate inorganic mineral component comprises P-tricalcium phosphate and/or hydroxyapatite and wherein the P-tricalcium phosphate and/or hydroxyapatite has a particle size of from about 5 microns to about 2500 microns.

5. The bone repair composition of any one of claims 1 to 4, wherein the first component and/or the second component further comprises:
i) one or more plasticizers, preferably wherein the one or more plasticizers comprise polyethylene glycol diesters of stearic acid, petrolatum, white petrolatum, lanolin, PEG-6000 distearate, PEG- 150 distearate, triacetin, poly(trimethylenecarbonate) (PTMC), polymethylmetacrylate (PMMA), polyvinylpyrrolidone (PVP), succinic anhydride, maleic anhydride, phthalic anhydride, or combinations thereof;
ii) one or more hydrocarbons, preferably wherein the one or more hydrocarbons comprise petrolatum, white petrolatum, or a combination thereof; and/or
iii) one or more carbonates, preferably wherein the one or more carbonates comprise poly(trimethylene carbonate), calcium carbonate, magnesium carbonate, aluminum carbonate, iron carbonate, zinc carbonate, calcium bicarbonate, sodium bicarbonate, magnesium bicarbonate, aluminum bicarbonate, iron bicarbonate, zinc bicarbonate, or combinations thereof.

6. The bone repair composition of any one of claims 1 to 5, wherein the first component and/or the second component further comprises one or more porogens and/or one or more chain extenders.

7. The bone repair composition of any one of claims 1 to 6, wherein the first component and/or the second component further comprises one or more antibiotics and/or one or more growth factors.

8. The bone repair composition of any one of claims 1 to 7, wherein the first component and/or the second component further comprises a colorant,
preferably wherein the first component comprises a colorant, preferably wherein the second component does not comprise a colorant or comprises a second colorant that is different than the first colorant; or
preferably wherein the second component comprises a colorant, preferably wherein the first component does not comprise a colorant or comprises a second colorant that is different than the first colorant;
preferably wherein the colorant comprises D&C Violet No. 2, D&C Blue No. 6, and/or D&C Green No. 6,
wherein the colorant comprises about 0.0001% to about 0.01% w/w of the total amount of the first component, the total amount of the second component, the total amount of the first component and the total amount of the second component, or the total amount of the combination of the first component and the second component.

9. The bone repair composition of any one of claims 1 to 8, wherein the bone repair composition is anhydrous and/or sterile

10. The bone repair composition of any one of the preceding claims for use in a method of repairing or reconstructing a bone defect in a subject, the method comprising:
(a) mixing the first component and the second component of the bone repair composition to form a polythiourethane, and
(b) administering the resulting polythiourethane to the bone defect,
preferably wherein the bone defect is a bone void, bone fracture, bleeding bone, or bone defect resulting from trauma, injury, infection, malignancy, and/or developmental malformation.

11. A kit comprising the bone repair composition of any one of claims 1-9,
preferably wherein the first component and second component are individually sealed in pouches or containers, optionally with vacuum sealing.

12. A method of making a two-component bone repair composition, the method comprising:
mixing the first component and the second component of the bone repair composition of any one of claims 1-9 together to form a polythiourethane,
preferably wherein the weight ratio of the first component to the second component is about 0.5:1 to about 5:1.

## Patentansprüche

1. Knochenreparaturzusammensetzung, umfassend zwei separate reaktive Komponenten, die miteinander gemischt werden können, um ein Polythiourethan zu bilden, wobei:
i) die erste Komponente Folgendes umfasst:
35 bis 45 Gew.-% eines isocyanatterminierten Präpolymers, umfassend das Reaktionsprodukt eines Überschusses von Hexamethylendiisocyanat (HDI) und/oder Isophorondiisocyanat (IPDI) mit Pentaerythrit (einem Polyol) unter Verwendung von Dibutylzinndilaurat als Katalysator;
1 bis 10 Gew.-% Pentaerythrittetrakis(3-mercaptopropionat);
0,00001 bis 0,01 Gew.-% Tributylamin;
0,1 bis 5 Gew.-% weißes Petrolatum; und
50 bis 60 Gew.-% β-Tricalciumphosphat; und
die zweite Komponente Folgendes umfasst:
1 bis 10 Gew.-% Isophorondiisocyanat;
30 bis 40 Gew.-% Pentaerythrittetrakis(3-mercaptopropionat);
0,00001 bis 0,01 Gew.-% Tributylamin;
1 bis 10 Gew.-% weißes Petrolatum; und
50 bis 60 Gew.-% β-Tricalciumphosphat;
ii) die erste Komponente Folgendes umfasst:
35 bis 45 Gew.-% eines isocyanatterminierten Präpolymers, umfassend das Reaktionsprodukt eines Überschusses von Hexamethylendiisocyanat (HDI) und/oder Isophorondiisocyanat (IPDI) mit Pentaerythrit (einem Polyol) unter Verwendung von Dibutylzinndilaurat als Katalysator;
1 bis 10 Gew.-% Pentaerythrittetrakis(3-mercaptopropionat);
0,00001 bis 0,01 Gew.-% Tributylamin;
0,1 bis 5 Gew.-% weißes Petrolatum; und
48 bis 58 Gew.-% β-Tricalciumphosphat; und
die zweite Komponente Folgendes umfasst:
2,5 bis 12,5 Gew.-% eines isocyanatterminierten Präpolymers, umfassend das Reaktionsprodukt eines Überschusses von Hexamethylendiisocyanat (HDI) und/oder Isophorondiisocyanat (IPDI) mit Pentaerythrit (einem Polyol) unter Verwendung von Dibutylzinndilaurat als Katalysator;
35 bis 45 Gew.-% Pentaerythrittetrakis(3-mercaptopropionat);
0,0001 bis 0,1 Gew.-% Tributylamin;
1 bis 10 Gew.-% weißes Petrolatum; und
45 bis 55 Gew.-% β-Tricalciumphosphat; oder
iii) die erste Komponente Folgendes umfasst:
30 bis 40 Gew.-% eines isocyanatterminierten Präpolymers, umfassend das Reaktionsprodukt eines Überschusses von Hexamethylendiisocyanat (HDI) und/oder Isophorondiisocyanat (IPDI) mit Pentaerythrit (einem Polyol) unter Verwendung von Dibutylzinndilaurat als Katalysator;
1 bis 10 Gew.-% Trimethylolpropantris(3-mercaptopropionat);
0,00001 bis 0,01 Gew.-% Tributylamin;
0,1 bis 5 Gew.-% weißes Petrolatum; und
50 bis 60 Gew.-% Hydroxylapatit; und
die zweite Komponente Folgendes umfasst:
1 bis 10 Gew.-% Isophorondiisocyanat;
30 bis 40 Gew.-% Pentaerythrittetrakis(3-mercaptopropionat);
0,00001 bis 0,01 Gew.-% Tributylamin;
1 bis 10 Gew.-% weißes Petrolatum; und
50 bis 60 Gew.-% β-Tricalciumphosphat.

2. Knochenreparaturzusammensetzung nach Anspruch 1, wobei die erste Komponente und/oder die zweite Komponente formbare Kitte sind, vorzugsweise wobei beim Mischen der ersten Komponente und der zweiten Komponente das resultierende Polythiourethan vor einem Aushärten ein formbarer Kitt ist, vorzugsweise wobei das resultierende Polythiourethan zur Verwendung als ein Knochenzement, ein Knochenklebstoff, ein Knochenhohlraumfüller, ein Knochentransplantatersatz und/oder ein blutstillendes Mittel für Knochen geeignet ist.

3. Knochenreparaturzusammensetzung nach einem der Ansprüche 1 bis 2, wobei die erste Komponente und/oder die zweite Komponente ferner einen oder mehrere Katalysatoren umfasst,
vorzugsweise wobei der eine oder die mehreren Katalysatoren Triethylamin, Tributylamin, 1,8-Diazabicyclo[5.4.0]undec-7-en, Dibutylzinndilaurat, Zinn(II)-octoat, 1,4-Diazabicyclo[2.2.2]octan, Tetra-n-butyltitanat, Titanacetylacetonat, Triethanolamintitanat, Titanethylacetoacetat, Tetraethyltitanat, Tetraisopropyltitanat, Titanmilchsäurechelat, Triethylendiamin, Bis(dimethylaminoethyl)ether, Dimethylethanolamin oder Kombinationen davon umfassen.

4. Knochenreparaturzusammensetzung nach einem der Ansprüche 1 bis 3, wobei die erste Komponente und/oder die zweite Komponente ferner eine oder mehrere partikelförmige anorganische Mineralverbindungen umfasst,
vorzugsweise wobei die eine oder die mehreren partikelförmigen anorganischen Mineralverbindungen Aragonit, Dahllit, Calcit, Calciumcarbonat, amorphes Calciumcarbonat, Vaterit, Weddellit, Whewellit, Struvit, Urat, Ferrihydrit, Francolith, Monohydrocalcit, Magnetit, Goethit, Dentin, Calciumcarbonat, Calciumsulfat, Calciumphosphosilicat, Natriumphosphat, Calciumaluminat, Calciumphosphat, Hydroxylapatit, a-Tricalciumphosphat, P-Tricalciumphosphat, Dicalciumphosphat, 3-Tricalciumphosphat, Tetracalciumphosphat, amorphes Calciumphosphat, Octacalciumphosphat, Calciumphosphatsilicaglas, Fluorapatit, Chlorapatit, magnesiumsubstituiertes Tricalciumphosphat, Magnesiumoxid, Magnesiumkaliumphosphat, Silica, Siliciumdioxid, lösliches Glas, Natriumsilicat, Carbonathydroxylapatit oder Kombinationen davon umfassen,
vorzugsweise wobei die partikelförmige anorganische Mineralkomponente P-Tricalciumphosphat und/oder Hydroxylapatit umfasst und wobei das P-Tricalciumphosphat und/oder Hydroxylapatit eine Partikelgröße von etwa 5 Mikrometer bis etwa 2500 Mikrometer aufweist.

5. Knochenreparaturzusammensetzung nach einem der Ansprüche 1 bis 4, wobei die erste Komponente und/oder die zweite Komponente ferner Folgendes umfasst:
i) einen oder mehrere Weichmacher, vorzugsweise wobei der eine oder die mehreren Weichmacher Polyethylenglykoldiester von Stearinsäure, Petrolatum, weißes Petrolatum, Lanolin, PEG-6000-Distearat, PEG-150-Distearat, Triacetin, Poly(trimethylencarbonat) (PTMC), Polymethylmethacrylat (PMMA), Polyvinylpyrrolidon (PVP), Bernsteinsäureanhydrid, Maleinsäureanhydrid, Phthalsäureanhydrid oder Kombinationen davon umfassen;
ii) einen oder mehrere Kohlenwasserstoffe, vorzugsweise wobei der eine oder die mehreren Kohlenwasserstoffe Petrolatum, weißes Petrolatum oder eine Kombination davon umfassen; und/oder
iii) ein oder mehrere Carbonate, vorzugsweise wobei das eine oder die mehreren Carbonate Poly(trimethylencarbonat), Calciumcarbonat, Magnesiumcarbonat, Aluminiumcarbonat, Eisencarbonat, Zinkcarbonat, Calciumbicarbonat, Natriumbicarbonat, Magnesiumbicarbonat, Aluminiumbicarbonat, Eisenbicarbonat, Zinkbicarbonat oder Kombinationen davon umfassen.

6. Knochenreparaturzusammensetzung nach einem der Ansprüche 1 bis 5, wobei die erste Komponente und/oder die zweite Komponente ferner ein oder mehrere Porogene und/oder einen oder mehrere Kettenverlängerer umfasst.

7. Knochenreparaturzusammensetzung nach einem der Ansprüche 1 bis 6, wobei die erste Komponente und/oder die zweite Komponente ferner ein oder mehrere Antibiotika und/oder einen oder mehrere Wachstumsfaktoren umfasst.

8. Knochenreparaturzusammensetzung nach einem der Ansprüche 1 bis 7, wobei die erste Komponente und/oder die zweite Komponente ferner einen Farbstoff umfasst,
vorzugsweise wobei die erste Komponente einen Farbstoff umfasst, vorzugsweise wobei die zweite Komponente keinen Farbstoff umfasst oder einen zweiten Farbstoff umfasst, der von dem ersten Farbstoff verschieden ist; oder
vorzugsweise wobei die zweite Komponente einen Farbstoff umfasst, vorzugsweise wobei die erste Komponente keinen Farbstoff umfasst oder einen zweiten Farbstoff umfasst, der von dem ersten Farbstoff verschieden ist;
vorzugsweise wobei der Farbstoff D&C Violet No. 2, D&C Blue No. 6 und/oder D&C Green No. 6 umfasst,
wobei der Farbstoff etwa 0,0001 Gew.-% bis etwa 0,01 Gew.-% der Gesamtmenge der ersten Komponente, der Gesamtmenge der zweiten Komponente, der Gesamtmenge der ersten Komponente und der Gesamtmenge der zweiten Komponente oder der Gesamtmenge der Kombination der ersten Komponente und der zweiten Komponente ausmacht.

9. Knochenreparaturzusammensetzung nach einem der Ansprüche 1 bis 8, wobei die Knochenreparaturzusammensetzung wasserfrei und/oder steril ist.

10. Knochenreparaturzusammensetzung nach einem der vorhergehenden Ansprüche zur Verwendung in einem Verfahren zum Reparieren oder Rekonstruieren eines Knochendefekts bei einem Subjekt, wobei das Verfahren Folgendes umfasst:
(a) Mischen der ersten Komponente und der zweiten Komponente der Knochenreparaturzusammensetzung, um ein Polythiourethan zu bilden, und
(b) Verabreichen des resultierenden Polythiourethans an den Knochendefekt,
vorzugsweise wobei der Knochendefekt ein Knochenhohlraum, eine Knochenfraktur, ein blutender Knochen oder ein Knochendefekt ist, der aus Trauma, Verletzung, Infektion, Malignität und/oder Entwicklungsfehlbildung resultiert.

11. Kit, umfassend die Knochenreparaturzusammensetzung nach einem der Ansprüche 1-9,
vorzugsweise wobei die erste Komponente und die zweite Komponente einzeln in Beuteln oder Behältern abgedichtet sind, optional mit Vakuumversiegelung.

12. Verfahren zum Herstellen einer Zweikomponenten-Knochenreparaturzusammensetzung, wobei das Verfahren Folgendes umfasst:
Mischen der ersten Komponente und der zweiten Komponente der Knochenreparaturzusammensetzung nach einem der Ansprüche 1-9, um ein Polythiourethan zu bilden,
vorzugsweise wobei das Gewichtsverhältnis der ersten Komponente zu der zweiten Komponente etwa 0,5:1 bis etwa 5:1 beträgt.

## Revendications

1. Composition de réparation osseuse comprenant deux composants réactifs distincts qui peuvent être mélangés ensemble pour former un polythiouréthane, dans laquelle :
i) le premier composant comprend :
35 à 45 % en poids d'un prépolymère à terminaison isocyanate comprenant le produit de réaction d'un excès de diisocyanate d'hexaméthylène (HDI) et/ou de diisocyanate d'isophorone (IPDI) avec du pentaérythritol (un polyol) à l'aide de dilaurate de dibutylétain comme catalyseur ;
1 à 10 % en poids de tétrakis(3-mercaptopropionate) de pentaérythritol ;
0,00001 à 0,01 % en poids de tributylamine ;
0,1 à 5 % en poids de vaseline blanche ; et
50 à 60 % en poids de β-phosphate tricalcique ; et
le second composant comprend :
1 à 10 % en poids de diisocyanate d'isophorone ;
30 à 40 % en poids de tétrakis(3-mercaptopropionate) de pentaérythritol ;
0,00001 à 0,01 % en poids de tributylamine ;
1 à 10 % en poids de vaseline blanche ; et
50 à 60 % en poids de β-phosphate tricalcique ;
ii) le premier composant comprend :
35 à 45 % en poids d'un prépolymère à terminaison isocyanate comprenant le produit de réaction d'un excès de diisocyanate d'hexaméthylène (HDI) et/ou de diisocyanate d'isophorone (IPDI) avec du pentaérythritol (un polyol) à l'aide de dilaurate de dibutylétain comme catalyseur ;
1 à 10 % en poids de tétrakis(3-mercaptopropionate) de pentaérythritol ;
0,00001 à 0,01 % en poids de tributylamine ;
0,1 à 5 % en poids de vaseline blanche ; et
48 à 58 % en poids de β-phosphate tricalcique ; et
le second composant comprend :
2,5 à 12,5 % en poids d'un prépolymère à terminaison isocyanate comprenant le produit de réaction d'un excès de diisocyanate d'hexaméthylène (HDI) et/ou de diisocyanate d'isophorone (IPDI) avec du pentaérythritol (un polyol) à l'aide de dilaurate de dibutylétain comme catalyseur ;
35 à 45 % en poids de tétrakis(3-mercaptopropionate) de pentaérythritol ;
0,0001 à 0,1 % en poids de tributylamine ;
1 à 10 % en poids de vaseline blanche ; et
45 à 55 % en poids de β-phosphate tricalcique ; ou
iii) le premier composant comprend :
30 à 40 % en poids d'un prépolymère à terminaison isocyanate comprenant le produit de réaction d'un excès de diisocyanate d'hexaméthylène (HDI) et/ou de diisocyanate d'isophorone (IPDI) avec du pentaérythritol (un polyol) à l'aide de dilaurate de dibutylétain comme catalyseur ;
1 à 10 % en poids de triméthylolpropane tris(3-mercaptopropionate) ;
0,00001 à 0,01 % en poids de tributylamine ;
0,1 à 5 % en poids de vaseline blanche ; et
50 à 60 % en poids d'hydroxyapatite ; et
le second composant comprend :
1 à 10 % en poids de diisocyanate d'isophorone ;
30 à 40 % en poids de tétrakis(3-mercaptopropionate) de pentaérythritol ;
0,00001 à 0,01 % en poids de tributylamine ;
1 à 10 % en poids de vaseline blanche ; et
50 à 60 % en poids de β-phosphate tricalcique.

2. Composition de réparation osseuse selon la revendication 1, dans laquelle le premier composant et/ou le second composant sont des pâtes modelables, de préférence dans laquelle, après mélange du premier composant et du second composant, le polythiouréthane résultant est une pâte modelable avant durcissement, de préférence dans laquelle le polythiouréthane résultant est adapté à une utilisation comme ciment osseux, adhésif osseux, matériau de remplissage des cavités osseuses, substitut de greffe osseuse et/ou agent hémostatique osseux.

3. Composition de réparation osseuse selon l'une quelconque des revendications 1 à 2, dans laquelle le premier composant et/ou le second composant comprennent en outre un ou plusieurs catalyseurs,
de préférence dans laquelle les un ou plusieurs catalyseurs comprennent la triéthylamine, la tributylamine, le 1,8-diazabicyclo[5.4.0]undéc-7-ène, le dilaurate de dibutylétain, l'octoate stanneux, le 1,4-diazabicyclo[2.2.2]octane, le titanate de tétra-n-butyle, l'acétylacétonate de titane, le titanate de triéthanolamine, l'éthylacétoacétate de titane, le titanate de tétraéthyle, le titanate de tétraisopropyle, le chélate d'acide lactique de titane, la triéthylènediamine, le bis(diméthylaminoéthyl)éther, la diméthyléthanolamine ou des combinaisons de ceux-ci.

4. Composition de réparation osseuse selon l'une quelconque des revendications 1 à 3, dans laquelle le premier composant et/ou le second composant comprennent en outre un ou plusieurs composés minéraux inorganiques particulaires,
de préférence dans laquelle les un ou plusieurs composés minéraux inorganiques particulaires comprennent l'aragonite, la dahlite, la calcite, le carbonate de calcium, le carbonate de calcium amorphe, la vatérite, la weddellite, la whewellite, la struvite, l'urate, la ferrihydrite, la francolite, la monohydrocalcite, la magnétite, la goethite, la dentine, le carbonate de calcium, le sulfate de calcium, le phosphosilicate de calcium, le phosphate de sodium, l'aluminate de calcium, le phosphate de calcium, l'hydroxyapatite, le phosphate tricalcique a, le phosphate tricalcique P, le phosphate dicalcique, le phosphate tricalcique 3, le phosphate tétracalcique, le phosphate de calcium amorphe, le phosphate octacalcique, le verre de silice au phosphate de calcium, la fluoroapatite, la chloroapatite, le phosphate tricalcique substitué au magnésium, l'oxyde de magnésium, le phosphate de magnésium et de potassium, la silice, le dioxyde de silicium, le verre soluble, le silicate de sodium, l'hydroxyapatite carbonatée ou des combinaisons de ceux-ci,
de préférence dans laquelle le composant minéral inorganique particulaire comprend du phosphate tricalcique P et/ou de l'hydroxyapatite et dans laquelle le phosphate tricalcique P et/ou l'hydroxyapatite ont une taille de particules d'environ 5 microns à environ 2 500 microns.

5. Composition de réparation osseuse selon l'une quelconque des revendications 1 à 4, dans laquelle le premier composant et/ou le second composant comprennent en outre :
i) un ou plusieurs plastifiants, de préférence dans laquelle les un ou plusieurs plastifiants comprennent des diesters de polyéthylène glycol d'acide stéarique, de la vaseline, de la vaseline blanche, de la lanoline, du distéarate de PEG-6000, du distéarate de PEG-150, de la triacétine, du poly(triméthylènecarbonate) (PTMC), du polyméthacrylate de méthyle (PMMA), de la polyvinylpyrrolidone (PVP), de l'anhydride succinique, de l'anhydride maléique, de l'anhydride phtalique ou des combinaisons de ceux-ci ;
ii) un ou plusieurs hydrocarbures, de préférence dans laquelle les un ou plusieurs hydrocarbures comprennent de la vaseline, de la vaseline blanche ou une combinaison de ceux-ci ; et/ou
iii) un ou plusieurs carbonates, de préférence dans laquelle les un ou plusieurs carbonates comprennent du poly(carbonate de triméthylène), du carbonate de calcium, du carbonate de magnésium, du carbonate d'aluminium, du carbonate de fer, du carbonate de zinc, du bicarbonate de calcium, du bicarbonate de sodium, du bicarbonate de magnésium, du bicarbonate d'aluminium, du bicarbonate de fer, du bicarbonate de zinc ou des combinaisons ceux-ci.

6. Composition de réparation osseuse selon l'une quelconque des revendications 1 à 5, dans laquelle le premier composant et/ou le second composant comprennent en outre un ou plusieurs porogènes et/ou un ou plusieurs extenseurs de chaîne.

7. Composition de réparation osseuse selon l'une quelconque des revendications 1 à 6, dans laquelle le premier composant et/ou le second composant comprennent en outre un ou plusieurs antibiotiques et/ou un ou plusieurs facteurs de croissance.

8. Composition de réparation osseuse selon l'une quelconque des revendications 1 à 7, dans laquelle le premier composant et/ou le second composant comprennent en outre un colorant,
de préférence dans laquelle le premier composant comprend un colorant, de préférence dans laquelle le second composant ne comprend pas de colorant ou comprend un second colorant qui est différent du premier colorant ; ou
de préférence dans laquelle le second composant comprend un colorant, de préférence dans laquelle le premier composant ne comprend pas de colorant ou comprend un second colorant qui est différent du premier colorant ;
de préférence dans laquelle le colorant comprend le violet D&C n° 2, le bleu D&C n° 6 et/ou le vert D&C n° 6,
dans laquelle le colorant comprend environ 0,0001 % à environ 0,01 % en poids de la quantité totale du premier composant, de la quantité totale du second composant, de la quantité totale du premier composant et de la quantité totale du second composant, ou de la quantité totale de la combinaison du premier composant et du second composant.

9. Composition de réparation osseuse selon l'une quelconque des revendications 1 à 8, dans laquelle la composition de réparation osseuse est anhydre et/ou stérile.

10. Composition de réparation osseuse selon l'une quelconque des revendications précédentes pour une utilisation dans un procédé de réparation ou de reconstruction d'un défaut osseux chez un sujet, le procédé comprenant :
(a) le mélange du premier composant et du second composant de la composition de réparation osseuse pour former un polythiouréthane, et
(b) l'administration du polythiouréthane résultant au défaut osseux,
de préférence dans laquelle le défaut osseux est une cavité osseuse, une fracture osseuse, un saignement osseux ou un défaut osseux résultant d'un traumatisme, d'une blessure, d'une infection, d'une tumeur maligne et/ou d'une malformation du développement.

11. Kit comprenant la composition de réparation osseuse selon l'une quelconque des revendications 1 à 9,
de préférence dans lequel le premier composant et le second composant sont scellés individuellement dans des sachets ou des contenants, éventuellement par scellage sous vide.

12. Procédé de fabrication d'une composition de réparation osseuse à deux composants, le procédé comprenant :
le mélange du premier composant et du second composant de la composition de réparation osseuse selon l'une quelconque des revendications 1 à 9 ensemble pour former un polythiouréthane, de préférence dans lequel le rapport pondéral du premier composant au second composant est d'environ 0,5:1 à environ 5:1.
